# EUROPEAN PATENT APPLICATION

(11) **EP 2 422 817 A1**
(43) Date of publication of application: **29.02.2012**
(21) Application number: 10305844.2
(22) Date of filing: 29.07.2010
(51) Int. Cl.: A61K 47/48, A61P 43/00

(54) **Flavin-nucleic acid ligand conjugates**

(71) Applicant: INSERM (Institut National de la Santé et de la Recherche Médicale), 75013 Paris (FR)
(72) Inventor: Giovannangeli, Carine, 75231 Paris Cedex 05 (FR)
(74) Representative: Cabinet Plasseraud

(57) **Abstract**

The invention relates to flavin-NAL conjugates and to their applications in the field of genetic engineering. Depending on the nature of the flavin which is conjugated to the NAL, the conjugates according to the invention can be used either (1) as nucleases for specifically and efficiently cleaving target nucleic acid sequences in living cells, or (2) as carriers for specifically and efficiently addressing NALs, such as for example antisense oligonucleotides, inside the cells.

SEQUENCE LISTING

<110> INSERM (Institut National de la Santé Et de la Recherche
Médicale)

<120> FLAVIN-NUCLEIC ACID LIGAND CONJUGATES

<130> BEP100447EP

<160> 15

<170> PatentIn version 3.3

<210> 1
<211> 12
<212> DNA
<213> Artificial

<220>
<223> PNA

<400> 1
   tccccccttt tc 12

<210> 2
<211> 12
<212> DNA
<213> Artificial

<220>
<223> PNA

<400> 2
   tccccccttt tc 12

<210> 3
<211> 12
<212> DNA
<213> Artificial

<220>
<223> PNA

<400> 3
   tccccccttt tc 12

<210> 4
<211> 13
<212> DNA
<213> Artificial

<220>
<223> PNA

<400> 4
ttttcctctc cct 13

<210> 5
<211> 13
<212> DNA
<213> Artificial

<220>
<223> PNA

<400>  5
ttttcctctc cct                                                          13


<210>  6
<211>  13
<212>  DNA
<213>  Artificial

<220>
<223>  PNA

<400>  6
ttttcctctc cct                                                          13


<210>  7
<211>  12
<212>  DNA
<213>  Artificial

<220>
<223>  PNA

<400>  7
tctgacgatt ag                                                           12


<210>  8
<211>  12
<212>  DNA
<213>  Artificial

<220>
<223>  PNA

<400>  8
tctgacgatt ag                                                           12


<210>  9
<211>  12
<212>  DNA
<213>  Artificial

<220>
<223>  PNA

<400>  9
tctgacgatt ag                                                           12


<210>  10
<211>  18
<212>  DNA
<213>  Artificial

<220>
<223>  PNA

<400>  10
aaatttaggg ataacagg                                                     18


<210>  11
<211>  18
<212>  DNA
<213>  Artificial

<220>
<223>  LNA-DNA

<400>  11
cctgttatcc ctaaattt                                                    18


<210>  12
<211>  23
<212>  DNA
<213>  Artificial

<220>
<223>  LNA-DNA

<400>  12
   tcccttttcc ttccctcctc ttt                                               23


<210>  13
<211>  57
<212>  DNA
<213>  Artificial

<220>
<223>  target oligonucleotide

<400>  13
gcaaatttag ggataacagg cctattaagc cgtaacctgc tgagttactg ctaggtt         57


<210>  14
<211>  57
<212>  DNA
<213>  Artificial

<220>
<223>  target oligonucleotide

<400>  14
gtacaattac cctgttatcc ctaaatttgc ctctcccccc ttttctttta aagcttg          57


<210>  15
<211>  16
<212>  DNA
<213>  Human immunodeficiency virus type 1

<400>  15
aaaagaaaag ggggga                                                      16

## Description

### FIELD OF THE INVENTION

The invention relates to flavin-nucleic acid ligand conjugates (flavin-NAL conjugates) having numerous applications in the field of genetic engineering.

### BACKGROUND OF THE INVENTION

Whole genome sequences are being determined in a growing number of species and DNA sequence variations involved in disease or other important biological functions are being identified always faster.

There is therefore a huge need for highly efficient methods of controlled genome manipulation and expression in all fields of life sciences.

Use of antisense therapies for efficiently and precisely controlling the expression of genes in genetic disorders and infections has been proposed. When the genetic sequence of a particular gene is known to be causative of a particular disease, it is possible to synthesize a strand of nucleic acid (DNA, RNA or a chemical analogue) that will bind to the messenger RNA (mRNA) produced by that gene and inactivate it, effectively turning that gene down or "off". Antisense drugs are for instance being researched to treat cancers (including lung cancer, colorectal carcinoma, pancreatic carcinoma, malignant glioma and malignant melanoma), diabetes, Duchenne muscular dystrophy and diseases such as asthma and arthritis with an inflammatory component. However, most potential therapies have not yet produced significant clinical results, in particular because these molecules are poorly addressed to the target cells. There is thus a huge need for new means for effectively addressing and delivering antisense molecules to the targeted cells.

Efficient and precise reprogramming of the genome requires having nucleases highly specific to the target sequence of interest. Existing methods are inefficient in most biological systems. In contrast, when using a nuclease that makes a specific double strand break in the target sequence, it has recently been shown that rates of modification between 0.5 and 50% are readily obtained. Consequently, many applications of genome reprogramming can now be considered. Importantly, nucleases used in genome modification need to be highly specific in order to avoid unwanted off-target cleavage and unwanted toxicity. Each and every targeted genome sequence modification project therefore requires designing the corresponding nuclease. There is therefore a huge need for novel sequence-specific endonucleases.

There are currently three types of sequence-specific endonucleases that can be used for controlled genome modification: zinc finger nucleases, modified homing endonucleases and synthetic nucleases. Less than 30 protein-based nucleases have been published so far (Carroll D., Gene Ther 15 (2008) 1463-1468; Paques F. et al., Curr Gene Ther 7 (2007) 49-66), and methods for producing adequate nucleases are still time-consuming because they require extensive screening of protein libraries for identification and optimization (Porteus M., Methods Mol Biol 435 (2008) 47-61, Smith J. et al., Nucleic Acids Res 34 (2006) e149). In addition, the range of sequences that can be targeted by any given approach is likely limited. For example, isolation of zinc finger nucleases targeting randomly chosen sequences was disappointingly low and the success rate could be considerably improved by selecting (GXX)n sequences and additional criteria (Ramirez C.L. et al., Nat Methods 5 (2008) 374-375).

Synthetic nucleases consist in conjugates of a DNA cleaving molecule and a sequence-specific ligand targeting the sequence of interest.

It has been reported that sequence-specific targeting and damaging of nucleic acids can be achieved *in vitro* using oligo-2'-deoxyribonucleotides (ODN) or peptide nucleic acids (PNA) covalently attached to a reactive compound (e.g. transition-metal complexe, polyamine, photosensitizer).

Examples of artificial nucleases are for instance described by Zenkova, M.A., (2004) Artificial nucleases, in Hans Joachim, Gross (ed.), Nucleic Acids and Molecular Biology, Vol. 13, Springer-Verlag Berlin Heidelberg.

It has also been shown that a bipyridine linked to two hairpin-polyamide is able to induce sequence-specific DNA break in cells (Simon et al, Nucleic acids res. 3531-3538, 2008). Moreover it was reported that a conjugate of orthophenanthroline (OP) and a triplex-forming oligonucleotide (TFO) specific to a 19bp sequence from human chromosome 7 induces efficient targeted genome modification. OP is a metal chelator able to catalyze the formation of oxidizing species that cleave DNA (Bales B.C. et al., Nucleic Acids Res 33 (2005) 5371-5379). It was successfully used against an integrated reporter locus and the endogenous chromosome 7 target sequence in different human cell lines (Cannata F. et al. Proc Natl Acad Sci U S A 105 (2008) 9576-9581). TFOs (Triplex Forming Oligonucleotides) form a triple-helix on specific oligo-purine sequences through Hoogsteen bonds. Genome scanning showed that adequate oligo-purine are abundant in the human and mouse genomes (Behe M.J., Nucleic Acids Res 23 (1995) 689-695; Manor H. et al., J Mol Evol 27 (1988) 96-101). However, since TFOs only target and cleave DNA sequences comprising oligo-purine sequences, their applications are limited.

Other conjugates have been proposed, such as a PNA-flavin conjugate (Simon P. et al., Angew. Chem. Int. Ed. 2006, 45, 6859-6861), which has been shown to be capable of modifying an isolated complementary single-stranded oligonucleotide *in vitro* when purified E Coli flavin reductase is added to the reaction mixture; the DNA modifications that are induced can be converted to breaks by a very strong basic treatment with piperidine, i.e. in experimental conditions very far from the physiological conditions. However, whereas DNA modification by flavin-PNA conjugates has been reported *in vitro* after addition of flavin reductase to the reaction mixture, it has been reported that flavin-oligonucleotide conjugates, in which the oligonucleotide contains only phosphodiester linkages and unmodified deoxyriboses, were unable to induce cleavage of single or double-stranded DNA when E Coli flavin reductase was added to the reaction mixture (Cécile Dueymes, Synthesis of flavin- or deazaflavin-oligonucleotide conjugates. Study of hybridization with RNA or DNA complementary sequences and enzymatic reduction; thesis defense of January 12, 2001, Université Joseph Fourier of Grenoble I, France).

It was thus considered that flavin conjugates would not have any cleavage activity in the cells for different reasons:
- as mentionned above, flavin-oligonucleotide conjugates are totally inefficient *in vitro* for induction of direct cleavage;
- concerning flavin-PNA conjugates, they do not exhibit direct cleavage activity *in vitro* but only a limited DNA modifying activity in non physiological conditions in the presence of purified *E. Coli* flavin reductase;
- finally, in the latter case, the flavin-induced damaging activity requires addition of *E. Coli* flavin reductase enzyme and no structurally homologous enzyme of the latter is known in eukaryotic and mammalian cells.

Accordingly, it would thus be very useful to provide conjugates capable of targeting all types of sequences in the genome of living cells with a high specificity and efficiency, either for modifying the sequence of the genes, i.e. reprogramming the genome, or for modifying the level of expression of the genes in said cells.

### SUMMARY OF THE INVENTION

The invention relates to conjugates able to address nucleic acid ligands (NALs) in living cells. Indeed, the inventors have shown that, surprisingly, flavin-NAL conjugates are capable of addressing NALs inside living cells: cells contacted with the conjugates according to the invention are transfected with the conjugates and thus with the NALs. Moreover, the inventors have shown, in contrast with the teaching of the prior art, that, depending on the nature of the flavin which is conjugated to the NAL, the conjugates according to the invention can be used as nucleases for specifically and efficiently cleaving target nucleic acid sequences in living cells. Thus, depending on the nature of the flavin which is conjugated to the NAL, the conjugates according to the invention can be used either (1) as nucleases for specifically and efficiently cleaving target nucleic acid sequences in living cells, or (2) as carriers for specifically and efficiently addressing NALs, such as for example antisense oligonucleotides, inside the cells.

### DETAILED DESCRIPTION OF THE INVENTION

### Definitions

As used herein, "flavin" refers to a group of organic compounds based on pteridine, formed by the tricyclic heteronuclear organic ring isoalloxazine and derivatives thereof, such as for example lumichrome.

As used herein, a "TFO" refers to a Triplex-Forming Oligonucleotide. DNA triplexes are formed when a purine-rich DNA duplex binds a single-stranded oligonucleotide [triplex-forming oligonucleotide (TFO)], through specific major groove interactions. TFOs are typically (T,C), (T,G) or (G,A) oligonucleotides, composed of 5-30 nucleotides in length.

As used herein, "NAL" stands for "Nucleic Acid Ligand", i.e. for a ligand that binds a nucleic acid sequence, said sequence being either DNA (DNA ligand) or RNA (RNA ligand). According to the invention, the NAL is typically either:
- a single or double strand nucleic acid polymer comprising from 5 to 200 nucleotides or a synthetic pyrrole-imidazole polyamide composed of 2 to 15 units, or
- a DNA or RNA antisense oligonucleotide comprising from 5 to 200 nucleotides,
said NAL being capable of pairing with a target DNA or RNA sequence. A nucleic acid polymer according to the invention is composed either of DNA or RNA. Particularly, said nucleic acid polymer is a TFO.

In addition, said nucleic acid polymer or said DNA or RNA antisense oligonucleotide according to the invention may comprise one or several nucleic acid analogues in its sequence or be constituted exclusively of nucleic acid analogues. Said nucleic acid analogues are typically selected from the group comprising GNA (Glycol Nucleic Acid), LNA (Locked Nucleic Acid), TNA (Threose Nucleic Acid), Phosphorothioates, Propyne analogs, N3'-P5' phosphoroamidate, FANA (2'-fluoro-arabino nucleic acid), CeNA (Cyclohexene nucleic acid), tcDNA (Tricyclo-DNA), 2'O-methyl etc., or mixtures thereof.

The nucleic acid polymer or the DNA or RNA antisense oligonucleotide according to the invention may also be selected from artificially synthesized polymers similar to DNA or RNA, such as for example morpholino or PMO (Phosphorodiamidate Morpholino Oligo), PNA (Peptidic Nucleic Acid), 2'-5' linked oligonucleotides, etc. As used herein, a "synthetic pyrrole-imidazole polyamide", also called MGB-P in this patent, means a polymer of 1-methyl-1H-pyrrole, 1-methyl-1H-imidazole, and 3-hydroxy-1-methyl-1H-pyrrole or other derivatives which can recognize a DNA specific sequence and bind the double-strand via the formation of a hairpin (Dervan et al, Curr Med Chem Anticancer Agents, 2005, 373-387). Polyamides according to the invention are for instance described by White S. et al., Recognition of the four Watson-Crick base pairs in the DNA minor groove by synthetic ligands, Nature, 1998, Jan. 29; 391(6666):468-71.

As used herein, a "conjugate" refers to two products/compounds covalently linked together.

As used herein, "to conjugate" means to link two products/compounds together.

As used herein, a C₁-C₂₀ linear or branched and substituted or not alkyl refers to a C₁ C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉ or C₂₀ linear or branched and substituted or not alkyl. As used herein, "substituted alkyl" refers to any type of substitution, such as for example to chemical groups selected from the group consisting of -NH₂, -COOH, -CO-NH₂, -COO-NH₂, -COO-R₁₁, -CHO, -OH, =O, -O-NH₂, -O-R₁₁, and -CO-R₁₁, wherein R₁₁ represents a C₁, C₂, C₃, C₄, or C₅ linear or branched alkyl.

According to the invention, the number of "consecutive atoms" has to be calculated from the first atom of the linker to the last atom of the linker following the shorter chain of consecutive atoms. Indeed, if the linker contains cyclic or heterocyclic systems, the number of atoms has to be calculated by following the shorter chain of atoms between A and Y.

An example of numeration according to the invention is given in the formula hereinafter:

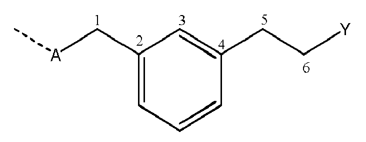

As shown in this formula, this linker separates A and Y by a chain of 6 consecutive atoms.

As used herein "a target DNA sequence" means either a coding or non-coding DNA sequence. In a particular embodiment, said target DNA sequence is a gene or a portion of a gene.

As used herein, "complementary to a significant portion of the sequence of the target DNA sequence" means that Y is complementary either to the whole sequence of said target DNA sequence or only to a portion of said target DNA sequence, said portion being specific of said target DNA sequence (i.e. said portion of said target DNA sequence is not comprised in a DNA sequence which is not targeted). Said portion of the target DNA sequence is typically a sequence of 10-30 base pairs, which is specific to the target DNA sequence.

As used herein, "complementary to a significant portion of the sequence of the RNA encoded by said gene" means that Y is complementary either to the whole sequence of said RNA or only to a portion of the sequence of said RNA, said portion being specific of the RNA which is targeted (i.e. said portion of the sequence of said RNA is not comprised in the sequence of a RNA which is not targeted). Said portion of the RNA is typically a sequence of 10-30 nucleotides, which is specific to the RNA which is targeted.

As used herein, "modifying the target DNA sequence" refers for instance to cleaving the DNA target sequence; deleting, substituting or adding one or more nucleotides in the sequence of said target DNA sequence, for instance if the target is a gene, for knocking-out said gene, deleting one or more exons of the sequence of a targeted gene (exon skipping); replacing partially or totally by homologous recombination the sequence of a DNA target by a sequence contained either in Y or in a "donor" DNA (represented for example by a plasmid, a PCR fragment or a ssDNA).

As used herein, a "cell" refers to any type of cells, either eukaryotic or prokaryotic, and also to unicellular organisms such as bacteria.

As used herein, "organism" refers to a human, an animal, a plant or a fungus.

As used herein, a "patient" refers to a human in need for a method according to the invention.

By "level of expression of a gene" it is meant the quantity of the functional gene product encoded by said gene that is produced by a cell. Typically, a gene product according to the invention is a protein (translated from the mRNA) or a functional RNA, such as for example a rRNA, a tRNA, a micro-RNA or any non-coding RNAs. According to the invention, the level of expression of a gene is considered to be modified when the difference between the level of expression of a gene in a cell which has been put into contact with a conjugate according to the invention and in a cell which has not been put into contact with said conjugate is statistically significant. Typically, said modification of the level of expression of genes is a statistically significant reduction of the level of expression of said genes, or the cancelation of the expression of said gene.

### Advantages of the conjugates according to the invention

The advantages of the conjugates according to the invention compared with protein-based nucleases, include rapid design and production. Moreover, surprisingly, the inventors have shown that the flavin-NAL conjugates according to the invention are capable of transfecting living cells and thereby delivering NALs into the cells. Still surprisingly, the inventors have shown that the flavin-NAL conjugates according to the invention are capable of delivering the NALs into the nucleus of the cells which are transfected. In addition, in contrast with the teaching of the prior art, the inventors have shown that when the flavin used in the conjugate has redox properties (i.e. can be reduced), the conjugate induces modifications in the genome sequence of living cells with a very high efficiency and selectivity. When the flavin derivative used in the conjugate has no redox properties, the conjugate can still deliver into the cells NALs such as antisense oligonucleotides which induce very selective and efficient decrease of the expression of the targeted gene.

In addition, contrary to the TFO-OP conjugates of the prior art, the flavin-NAL conjugates according to the invention can target any type of nucleic acid sequence of the genome. Indeed, since TFOs can only target oligopyrimidine•oligopurine sequences, the number of sequences that can be targeted by TFO-OP conjugates is limited. In contrast, since the NALs according to the invention are not limited to TFOs, the flavin-NAL conjugates of the invention can target any type of DNA or RNA sequence.

The conjugates according to the invention have therefore numerous applications in the field of genetic engineering, depending on the nature of the NAL and of the flavin that form the conjugate according to the invention. Examples of applications are gene targeting, i.e. methods in which a DNA molecule introduced into a cell replaces the corresponding chromosomal segment by homologous recombination; targeted mutagenesis, i.e. modification of targeted sequence resulting from incorrect repair of damaged DNA; inhibition or modification of the expression of a target gene in a sequence-specific manner (antisense technologies, exon-skipping); etc.

### Methods for transfecting cells with NALs

The invention relates to a method for transfecting a cell with a NAL, said method comprising the step of contacting said cell with a flavin-NAL conjugate.

The invention also relates to a flavin-NAL conjugate for use in a method for transfecting a cell with a NAL, said method comprising the step of contacting said cell with a flavin-NAL conjugate.

In one embodiment of the invention, said method for transfecting a cell with a NAL is carried out *in vitro.*

In another embodiment of the invention, said method for transfecting a cell with a NAL is carried out *ex vivo.* In this embodiment, the cell is obtained from a patient, contacted *ex vivo* with a conjugate according to the invention, and then reintroduced in the patient.

In one embodiment of the invention, said method for transfecting a cell with a NAL is carried out *in vivo.*

In a particular embodiment of the invention, said method for transfecting a cell with a NAL is carried out *in vivo* by direct microinjection into one-cell stage mouse or rat embryos.

In another embodiment, said flavin-NAL conjugate has the formula selected from the group consisting of formulae (I)-(XI) as defined hereinafter.

### Flavin-NAL conjugates according to the invention

The invention thus relates to a flavin-NAL conjugate having formula (I) 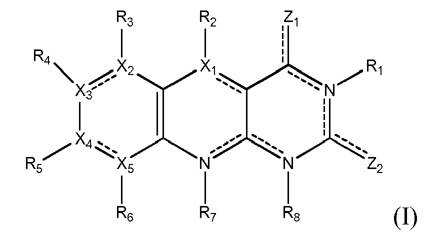 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂₀ linear or branched and substituted or not alkyl,
- Z₂ is selected from the group consisting of [-A-L-Y], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, -NH₂, -CF₃, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅, R₆, R₇ and R₈ are independently present or not and if present are independently selected from the group consisting of [-A-L-Y], H, F, Cl, Br, I, C₁-C₂₀ linear or branched and substituted or not alkyl, -NH₂, -CF₃, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
wherein only one of Z₂, R₅, R₆, R₇ and R₈ represents [-A-L-Y], and wherein
- A is selected from the group consisting of =CH-, -CH₂-, -O-, -S-, -NH-,
- L is a linker which separates A and Y by a chain of at least 3 consecutive atoms, and
- Y is a Nucleic Acid Ligand (NAL).

Typically,Y is as defined previously, i.e. is either:
- a single or double strand nucleic acid polymer comprising from 5 to 200 nucleotides or a synthetic pyrrole-imidazole polyamide composed of 2 to 15 units, or
- a DNA or RNA antisense oligonucleotide comprising from 5 to 200 nucleotides,
said NAL being capable of pairing with a target DNA or RNA sequence.

According to the invention, the chemical nature of the linker L is not relevant: all types of linkers ensuring a sufficient spacing between A and Y are suitable. Indeed, if the spacing between A and Y is not sufficient, the steric hindrance generated by the NAL nearby the flavin will cancel or decrease the redox activity of the flavin. The inventors have found that "sufficient spacing" between the A and Y is obtained when the linker separates A and Y by at least 3 consecutive atoms. Typically, the linker separates A and Y by at least 3, 4, 5, 6, 7, 8, 9, 10, 11, 12, 13, 14, 15, 16, 17, 18, 19, 20 consecutive atoms.

Typically, in the conjugates according to the invention, said linker L has the chemical structure -T₁-T₂- (i.e. [-A-T₁-T₂-Y]), wherein
- T₁ is a substituted or not, linear or branched alkyl or alkenyl chain, wherein one or more carbon atom of said chain are optionally replaced by a chemical group selected from the group consisting of ether (-O-), amine (-NH), thioether (-S-), cetone (-CO-), ester (-CO-O-), amide (-CO-NH-), urea (-NH-CO-NH-), thiourea (-NH-CS-NH-), oxycarbonyl (-O-CO-O-), carbamate (-NH-CO-O-), and cyclic or heterocyclic systems, said cyclic or heterocyclic systems being saturated or not and substituted or not, and
- T₂ is a chemical moiety selected from the group consisting of 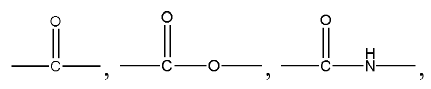 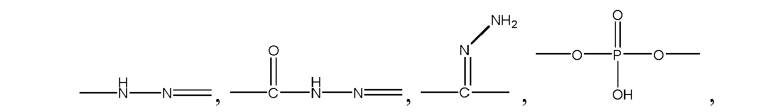 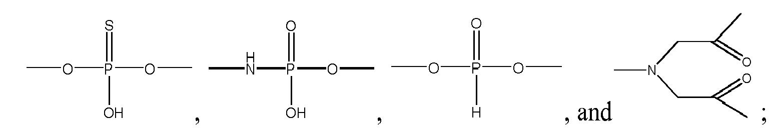 provided that said linker L separates the A and the Y moieties by at least 3 consecutive atoms.

In a particular embodiment, when T₂ is 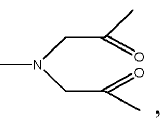 [-A-L-Y] has the following structure: 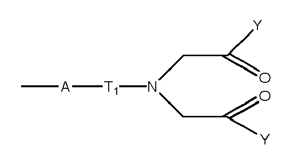 wherein A, T₁ and Y are as disclosed previously. In this particular embodiment, the conjugates according to the invention thus comprise two Y moieties linked on a same flavin derivative.

In a particular embodiment, said cyclic or heterocyclic systems of are selected from the group comprising azetidine, oxetane, thietane, pyrrole, furan, pyrroline, tetrahydrofuran, thiophene, tetrahydrothiophene, pyrazole, imidazole, oxazole, isoxazole, pyrazoline, imidazoline, pyrazolidine, imidazolidine, dioxolane, thiazole, isothiazole, thiazolidine, isoxazolidine, triazole, oxadiazole, furazan, thiadiazole, tetrazole, pyridine, naphthyridine, pyran, dihydro-pyran, piperidine, pyridazine, pyrimidine, purine, pyrazine, pteridine, oxazine, dioxine, piperazine, morpholine, dioxane, thiazine, thiomorpholine, oxathiane, dithiane, triazine, trioxane, thiadiazine, dithiazine, trithiane, cyclobutane, cylcobutene, cyclopentane, cyclopentene, cyclohexane, cyclohexene cycloheptane, cycloheptene, and benzene and its derivatives.

Examples of derivatives of benzene are indene, indane, indolizine, indole, benzofuran, indoline, benzothiophene, indazole, benzimidazole, benzthiazole, naphthalene, tetralin, quinoline, chromene, chromane, cinnoline, quinazoline, quinoxaline and phthalazine.

As previously mentioned, said alkyl or alkenyl chain and said cyclic or heterocyclic systems may be substituted, for instance by a C₁-C₁₀ alkyl, such as for example by a methyl, ethyl, propyl or isopropyl, or by functional groups such as for example by alcohol, amine, amide, ketone, aldehyde, ester, ether or acid functions, etc.

In a particular embodiment, L has one of the following formulae:

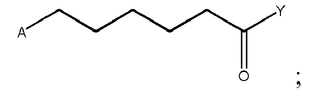

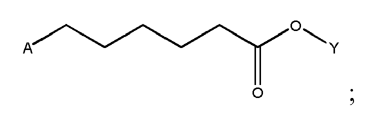

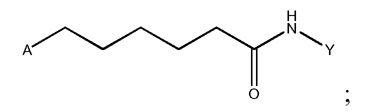

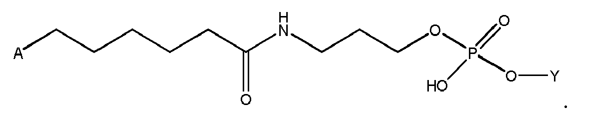

In one embodiment of the invention, the flavin-NAL conjugate according to the invention is not the conjugate having one of the formulae selected from the group consisting of: 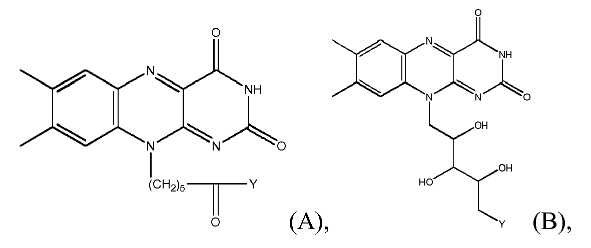 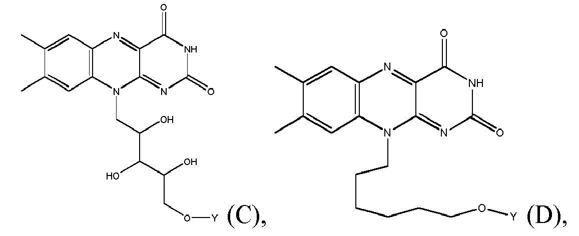 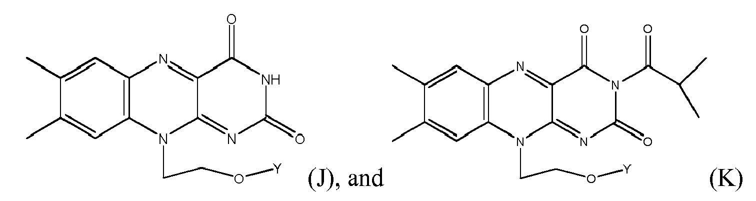 wherein Y is as defined previously.

As it will be easily understood by the skilled person, to enhance the cell penetration properties of the NALs, and/or to enhance the nuclease activity of the conjugates according to the invention, each NAL can be conjugated with more than one flavin moiety.

Moreover, in the conjugates according to the invention, it is possible that Y represents more than one NAL. Indeed, in some applications, it might be interesting to link two or more NALs to the same linker for enhancing the recognition of the target gene by the conjugate.

Typically, depending on the nature of the flavin which is conjugated to the NAL, the conjugates according to the invention can be used either as (1) nucleases for specifically and efficiently cleaving targeted genes in living cells, or (2) as carriers for specifically and efficiently addressing NALs, such as for example antisense oligonucleotides, inside the cells.

### Nucleases

It has been shown that the redox activity of flavin requires that the isoalloxazine ring be substituted in position 10 either by a [-CH₂-L-Y] moiety or by an alkyl. As a result, the conjugates wherein the flavin is substituted in position 10 by a [-CH₂-L-Y] moiety or by an alkyl will have a nuclease activity. Accordingly, the conjugates according to the invention having formulae (II)-(IX), as defined hereinafter are selective nucleases and have therefore numerous applications in the field of genetic engineering. Indeed, the inventors have shown that, when cells are put into contact with a conjugate according to the invention, the conjugates are internalized in said cells, delivered to the nucleus of said cells, and the flavin moiety of said conjugate induces for instance, in said cells, after redox reaction, a cleavage in the DNA sequence of the gene targeted by said NAL.

In one embodiment, the invention thus relates to a flavin-NAL conjugate having formula (II) 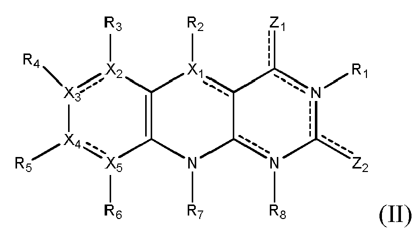 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂₀ linear or branched and substituted or not alkyl,
- Z₂ is selected from the group consisting of [-A-L-Y], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅, R₆, and R₈ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
- R₇ is selected from the group consisting of [-CH₂-L-Y] and C₁-C₂₀ linear or branched and substituted or not alkyl,
wherein only one of Z₂, R₅, R₆, R₇ and R₈ represents [-A-L-Y] or [-CH₂-L-Y], and wherein
o A is selected from the group consisting of =CH-, -CH₂-, -O-, -S-, - NH-,
o L is a linker which separates A and Y by a chain of at least 3 consecutive atoms, and
o Y is a NAL.

More particularly, the invention relates to a flavin-NAL conjugate having formula (III) 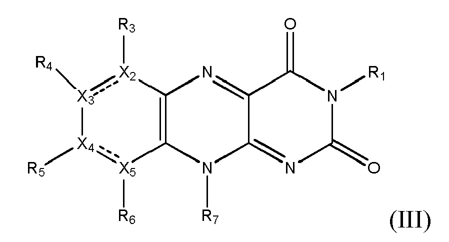 wherein
- the doted lines represent a bond which is present or not,
- X₂, X₃, X₄ and X₅ are independently selected from the group consisting of C, CH or N,
- R₁, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
- R₅ and R₆ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, - CHO, -OH, -O-R₉, and -CO-R₉, wherein R₉ and R₁₀ represent independently a C₁-C₂₀ linear or branched and substituted or not alkyl, and
- R₇ is selected from the group consisting of [-CH₂-L-Y] and C₁-C₂₀ linear or branched and substituted or not alkyl,
wherein only one of R₅, R₆ and R₇ represents [-A-L-Y] or [-CH₂-L-Y], and wherein
o A is selected from the group consisting of =CH-, -CH₂-, -O-, -S-, - NH-,
o L is a linker which separates A and Y by a chain of at least 3 consecutive atoms, and
o Y is a NAL.

Still particularly, the invention relates to a flavin-NAL conjugate having formula (IV) 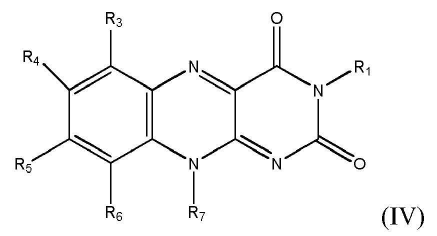 wherein
- R₁, R₃ and R₄ are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, - COO-NH₂, -COO-R₉, -CHO, -OH, -O-R₉, and -CO-R₉, wherein R₉ and R₁₀ represent independently a C₁-C₂₀ linear or branched and substituted or not alkyl,
- R₅ and R₆ are independently selected from the group consisting of [-A-L-Y], H, F, Cl, Br, I, C₁-C₂₀ linear or branched and substituted or not alkyl, -CF₃, - NH₂, -O-NH₂, -NHR₉, -O-NHR₉, -NR₉R₁₀, -O-NR₉R₁₀, -COOH, -CO-NH₂, - COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₇ is selected from the group consisting of [-CH₂-L-Y] and C₁-C₂₀ linear or branched and substituted or not alkyl,
wherein only one of R₅, R₆ and R₇ represents [-A-L-Y] or [-CH₂-L-Y], and wherein
o A is selected from the group consisting of =CH-, -CH₂-, -O-, -S-, - NH-,
o L is a linker which separates A and Y by a chain of at least 3 consecutive atoms, and
o Y is a NAL.

More particularly, the invention relates to a flavin-NAL conjugate having formula (V) 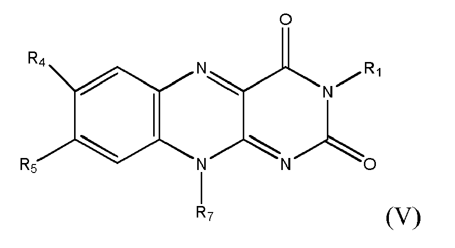 wherein
- R₁ and R₄ are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
- R₅ is selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and OK-CO-R₉, wherein R₉ and R₁₀ represent independently a C₁-C₂₀ linear or branched and substituted or not alkyl, and
- R₇ is selected from the group consisting of [-CH₂-L-Y] and C₁-C₂₀ linear or branched and substituted or not alkyl,
wherein only one of R₅ and R₇ represents [-A-L-Y] or [-CH₂-L-Y], and wherein
o A is selected from the group consisting of =CH-, -CH₂-, -O-, -S-, - NH-,
o L is a linker which separates A and Y by a chain of at least 3 consecutive atoms, and
o Y is a NAL.

Again particularly, the invention relates to a flavin-NAL conjugate having formula (VI) 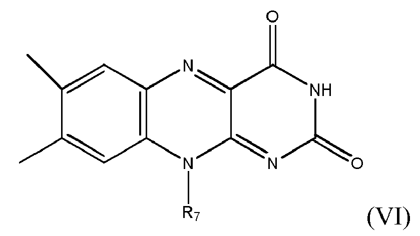 wherein R₇ is [-CH₂-L-Y], wherein
o L is a linker which separates A and Y by a chain of at least 3 consecutive atoms, and
o Y is a NAL.

Still particularly, the invention relates to a flavin-NAL conjugate having formula (VII) 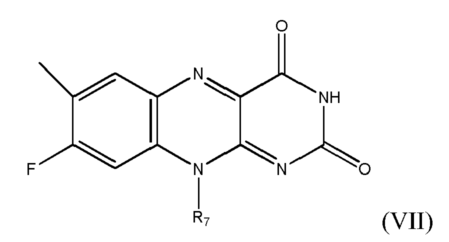 wherein R₇ is [-CH₂-L-Y], wherein
o L is a linker which separates A and Y by a chain of at least 3 consecutive atoms, and
o Y is a NAL.

Again particularly, the invention relates to a flavin-NAL conjugate having formula (VIII) 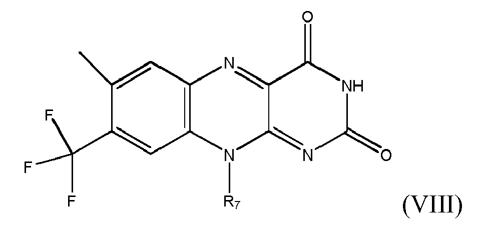 wherein R₇ is [-CH₂-L-Y], wherein
o L is a linker which separates A and Y by a chain of at least 3 consecutive atoms, and
o Y is a NAL.

Still particularly, the invention relates to a flavin-NAL conjugate having formula (IX) 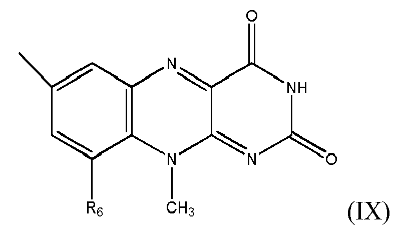 wherein R₆ represents [-A-L-Y], and wherein
o A is selected from the group consisting of =CH-, -CH₂-, -O-, -S-, - NH-,
o L is a linker which separates A and Y by a chain of at least 3 consecutive atoms, and
o Y is a NAL.

In the conjugates according to the invention having formulae (II)-(IX), L is as defined for formula (I).

### Nature of Y in conjugates (I)-(IX)

In the conjugates according to the invention having formulae (I)-(IX), and particularly in the conjugates according to the invention having formulae (II)-(IX), the NAL is typically selected from the group consisting of any NAL capable of targeting a RNA or a DNA sequence.

Typically, said NAL capable of targeting a RNA or a DNA sequence according to the invention is a NAL capable of pairing specifically with a RNA or a DNA sequence. Still typically, said NAL capable of pairing specifically with a RNA or a DNA sequence is an NAL which is complementary to said RNA or DNA sequence. In particular, said NAL is a single or double strand nucleic acid polymer comprising from 5 to 200 nucleotides or a synthetic pyrrole-imidazole polyamide composed of 2 to 15 units, said NAL being capable of pairing with a target DNA or RNA sequence, as defined previously in the definition section.

In a particular embodiment, the oligonucleotide is a TFO.

In another embodiment, said NAL is an oligonucleotide comprising Locked Nucleic Acids (LNAs) in its sequence. In still another particular embodiment, the oligonucleotide is composed only of LNAs. Particularly, said LNA is a TFO/LNA, i.e. a TFO composed exclusively or partially of locked nucleic acids.

In another embodiment, in the conjugates (I)-(IX), said NAL according to the invention is a Peptidic Nucleic Acid (PNA) oligonucleotide.

### Applications of conjugates (I)-(IX)

The invention also relates to the uses of the conjugates according to the invention. Typically, the conjugates having the formulae (I), (II), (III), (IV), (V), (VI), (VII), (VIII) and (IX) can be used either for modifying a target DNA sequence or for modifying the level of expression of a gene.

### Modification of a target DNA sequence

The invention thus relates to a method for modifying a target DNA sequence in a cell, said method comprising the step of contacting said cell with a sufficient amount of a flavin-NAL conjugate having the formulae (I), (II), (III), (IV), (V), (VI), (VII), (VIII) or (IX) wherein Y is complementary to a significant portion of the target DNA sequence.

The invention also relates to a flavin-NAL conjugate having the formulae (I), (II), (III), (IV), (V), (VI), (VII), (VIII) or (IX), for use in a method for modifying a target DNA sequence in a cell, said method comprising the step of contacting said cell with a sufficient amount of said flavin-NAL conjugate, wherein, in said flavin-NAL conjugate, Y is complementary to a significant portion of the target DNA sequence.

Particularly, the invention relates to a method for modifying a target DNA sequence in a cell *in vitro,* said method comprising the step of contacting said cell with a sufficient amount of a conjugate according to the invention selected from the group consisting of the conjugates having the formulae (I), (II), (III), (IV), (V), (VI), (VII), (VIII) and (IX) wherein Y is complementary to a significant portion of the target DNA sequence.

The invention also relates to a method for modifying a target DNA sequence in a cell *ex vivo,* said method comprising a step of contacting a cell obtained from a patient with a sufficient amount of a conjugate according to the invention selected from the group consisting of the conjugates having the formulae (I), (II), (III), (IV), (V), (VI), (VII), (VIII) and (IX) wherein Y is complementary to a significant portion of the target DNA sequence, and then a step of re-introducing in said patient the cell which has been contacted with said conjugate.

The invention still relates to the conjugates having the formulae (I), (II), (III), (IV), (V), (VI), (VII), (VIII) and (IX) for use in a method for modifying a target DNA sequence in a cell *ex vivo,* wherein Y is complementary to a significant portion of the target DNA sequence.

In the methods according to the invention, during said step of contacting said cell with a conjugate according to the invention, the internalization of the conjugate by the cell can be facilitated by any transfection method known by the skilled person, such as for example electroporation, microinjection, etc.

The invention also relates to a method for modifying a target DNA sequence in a cell *in vivo,* said method comprising the step of administering to an organism in which said target DNA sequence shall be modified, a sufficient amount of a conjugate according to the invention selected from the group consisting of the conjugates having the formulae (I), (II), (III), (IV), (V), (VI), (VII), (VIII) and (IX) wherein Y is complementary to a significant portion of said target DNA sequence. In a particular embodiment, said step of administering is performed by microinjection into one-cell embryo. In another particular embodiment, said step of administering is performed by microinjection into one-cell non-human embryo. The invention also relates to a conjugate according to the invention selected from the group consisting of the conjugates having the formulae (I), (II), (III), (IV), (V), (VI), (VII), (VIII) and (IX) for use in a method for modifying a target DNA sequence in a cell *in vivo,* wherein Y is complementary to a significant portion of said target DNA sequence.

In the methods for modifying a target DNA sequence according to the invention, said sufficient amount is an amount effective for inducing a cleavage in said target DNA sequence.

In a particular embodiment, in the methods for modifying a target DNA sequence according to the invention, donor nucleic acid can be introduced in cells treated with the flavin-NAL conjugates according to the invention in order to modify the target DNA sequence. Donor nucleic acid is typically single-stranded DNA or double-stranded DNA in the form of chemically synthesized oligonucleotide, PCR product, plasmid that will carry novel sequence information that will be introduced at the targeted locus.

Since the redox activity of the flavin in the cells is iron dependent (see experimental section), one embodiment of the invention relates to the methods for modifying a target DNA sequence according to the invention, wherein the conjugate is put into contact with the cells and/or administered together with ferric ammonium citrate (FAC), which has been shown to enhance the cleavage and mutagenic activities of the conjugates according to the invention. Any other method for increasing intracellular iron content may also be considered.

### Modification of the level of expression of a gene

The invention further relates to a method for modifying the level of expression of a gene in a cell, said method comprising the step of contacting said cell with a sufficient amount of a flavin-NAL conjugate having the formulae (I), (II), (III), (IV), (V), (VI), (VII), (VIII) or (IX) wherein Y is complementary to a significant portion of the sequence of the RNA encoded by said gene.

The invention thus relates to a flavin-NAL conjugate having the formulae (I), (II), (III), (IV), (V), (VI), (VII), (VIII) or (IX), for use in a method for modifying the level of expression of a gene in a cell, said method comprising the step of contacting said cell with a sufficient amount of said flavin-NAL conjugate, wherein, in said flavin-NAL conjugate, Y is complementary to a significant portion of the sequence of the RNA encoded by said gene.

The invention also relates to a method for modifying the level of expression of a gene in a cell *in vitro,* said method comprising the step of contacting said cell with sufficient amount of a conjugate according to the invention selected from the group consisting of the conjugates having the formulae (I), (II), (III), (IV), (V), (VI), (VII), (VIII) and (IX) wherein Y is complementary to a significant portion of the sequence of the RNA encoded by said gene. Indeed, thanks to the recognition of the RNA by the Y moiety of the conjugate, the redox activity of the flavin moiety of the conjugate induces a cleavage in the sequence of said RNA, thereby diminishing the level of expression of the gene or cancelling the expression of the gene.

According to the invention, said RNA encoded by said gene is either a mRNA, a functional RNA, such as for example a rRNA, a tRNA or a microRNA, or any non-coding RNAs.

In the method for modifying the level of expression of a gene according to the invention, during said step of contacting said cell with a conjugate according to the invention, the internalization of the conjugate by the cell can be facilitated by any transfection method known by the skilled person, such as for example electroporation, microinjection, etc.

The invention also relates to a method for modifying the level of expression of a gene in a cell *in vivo,* said method comprising the step of administering to an organism in which the expression of said gene shall be modified, a sufficient amount of a conjugate according to the invention selected from the group consisting of the conjugates having the formulae (I), (II), (III), (IV), (V), (VI), (VII), (VIII) and (IX) wherein Y is complementary to a significant portion of the sequence of the RNA encoded by said gene. Typically, said sufficient amount is an amount effective for modifying the expression of said gene.

The invention also relates to a conjugate according to the invention selected from the group consisting of the conjugates having the formulae (I), (II), (III), (IV), (V), (VI), (VII), (VIII) and (IX) for use in a method for modifying the level of expression of a gene in a cell *in vivo,* wherein Y is complementary to a significant portion of the sequence of the RNA encoded by said gene.

In a particular embodiment of the methods for modifying the target DNA sequence in a cell according to the invention, or of the methods for modifying the level of expression of a gene in a cell according to the invention, the conjugate which is used is a conjugate having one of the following formulae:

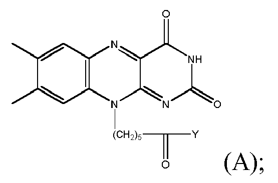

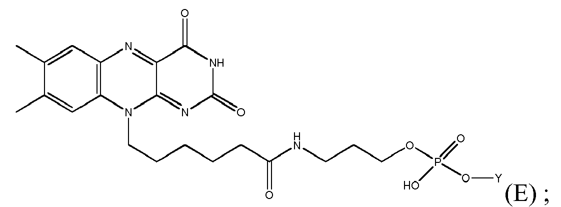

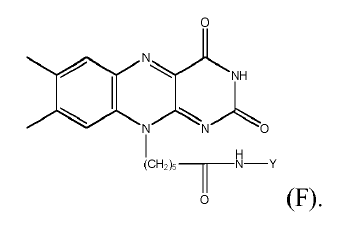

Accordingly, the conjugates according to the invention are useful either for research work or in therapy, for instance in gene therapy or for treating diseases wherein a gene is overexpressed.

### Carrier conjugates

It has been shown that flavin is not reduced in cells when the atom N in position 10 of the isoalloxazine ring is not substituted. These derivatives of flavin are called lumichrome. The inventors have shown that when the flavin moiety of the conjugates according to the invention is lumichrome or a derivative thereof, e.g. conjugates having formulae (X)-(XV) as defined hereinafter, these conjugates do not have any nuclease activity i.e. do not induce cleavages in the targeted sequences.

However, the inventors have found that lumichrome and derivatives thereof are very good carriers to address NALs inside cells

Therefore, the conjugates having formulae (X)-(XV) are very promising candidates for specifically delivering NALs in a cell, for instance antisense oligonucleotides for modifying the level of expression of genes.

Accordingly, in a particular embodiment, the invention relates to a flavin-NAL conjugate having formula (X) 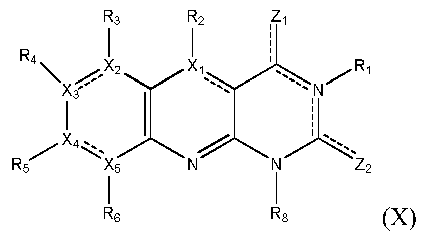 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂₀ linear or branched and substituted or not alkyl,
- Z₂ is selected from the group consisting of [-A-L-Y], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅, R₆ and R₈ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
wherein only one of Z₂, R₅, R₆ and R₈ represents [-A-L-Y], and wherein
- A is selected from the group consisting of =CH-, -CH₂-, -O-, -S-, -NH-,
- L is a linker which separates A and Y by a chain of at least 3 consecutive atoms, and
- Y is a Nucleic Acid Ligand (NAL).

More particularly, the invention relates to a flavin-NAL conjugate having formula (XI) 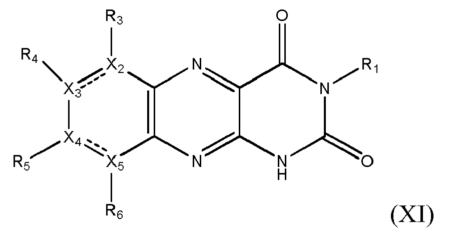 wherein
- the doted lines represent a bond which is present or not,
- X₂, X₃, X₄ and X₅ are independently selected from the group consisting of C, CH or N,
- R₁, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅ and R₆ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, - CHO, -OH, -O-R₉, and -CO-R₉, wherein R₉ and R₁₀ represent independently a C₁-C₂₀ linear or branched and substituted or not alkyl,
wherein only one of R₅ and R₆ represents [-A-L-Y], and wherein
- A is selected from the group consisting of =CH-, -CH₂-, -O-, -S-, -NH-,
- L is a linker which separates A and Y by a chain of at least 3 consecutive atoms, and
- Y is a Nucleic Acid Ligand (NAL).

Still particularly, the invention relates to a flavin-NAL conjugate having formula (XII) 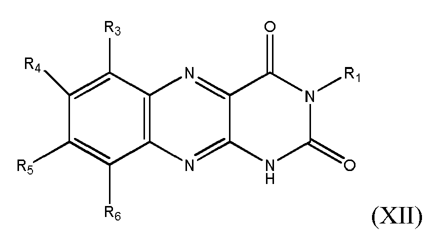 wherein
- R₁, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅ and R₆ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, - CHO, -OH, -O-R₉, and -CO-R₉, wherein R₉ and R₁₀ represent independently a C₁-C₂₀ linear or branched and substituted or not alkyl,
wherein only one of R₅ and R₆ represents [-A-L-Y], and wherein
- A is selected from the group consisting of =CH-, -CH₂-, -O-, -S-, -NH-,
- L is a linker which separates A and Y by a chain of at least 3 consecutive atoms, and
- Y is a Nucleic Acid Ligand (NAL).

More particularly, the invention relates to a flavin-NAL conjugate having formula (XIII) 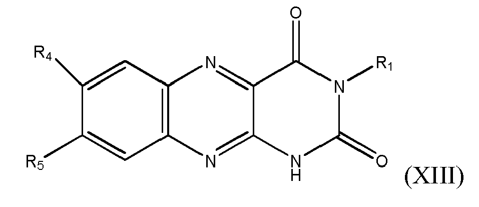 wherein
- R₁ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, - CHO, -OH, -O-R₉, and -CO-R₉, wherein R₉ and R₁₀ represent independently a C₁-C₂₀ linear or branched and substituted or not alkyl, and
- R₅ is [-A-L-Y], wherein
   o A is selected from the group consisting of =CH-, -CH₂-, -O-, -S-, - NH-,
   o L is a linker which separates A and Y by a chain of at least 3 consecutive atoms, and
   o Y is a Nucleic Acid Ligand (NAL).

Again particularly, the invention relates to a flavin-NAL conjugate having formula (XIV) 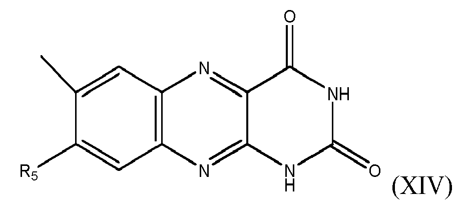 wherein R₅ is [-A-L-Y], wherein
o A is selected from the group consisting of =CH-, -CH₂-, -O-, -S-, - NH-,
o L is a linker which separates A and Y by a chain of at least 3 consecutive atoms, and
o Y is a Nucleic Acid Ligand (NAL).

Still particularly, the invention relates to a flavin-NAL conjugate having the formula (XV) 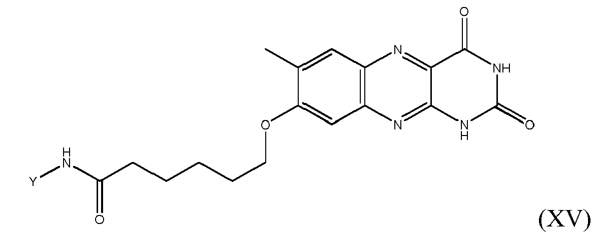 wherein Y is a Nucleic Acid Ligand (NAL).

In the conjugates having formulae (X)-(XV) according to the invention, L is as defined for formula (I).

### Nature of Y in conjugates (I) and (X)-(XV)

In the conjugates according to the invention having formulae (I) or (X)-(XV), and particularly in the conjugates according to the invention having formulae (X)-(XV), the NAL Y is typically selected from the group consisting of antisense oligonucleotides. According to the invention, an antisense oligonucleotide is an oligonucleotide capable of targeting a RNA sequence. In one embodiment, the NAL is an antisense oligonucleotide comprising from 5 to 200 nucleotides capable of pairing with a RNA target. In a particular embodiment, said NAL is an antisense oligonucleotide comprising from 5 to 50 nucleotides, particularly 15 to 25 nucleotides, capable of pairing with a RNA target. Said antisense oligonucleotide is composed either of DNA or RNA. In addition, said antisense oligonucleotide may comprise one or several nucleic acid analogues in its sequence or be constituted exclusively of nucleic acid analogues. Said nucleic acid analogues are typically selected from the group comprising GNA (Glycol Nucleic Acid), LNA (Locked Nucleic Acid), TNA (Threose Nucleic Acid), Phosphorothioates, Propyne analogs, N3'-P5' phosphoroamidate, FANA (2'-fluoro-arabino nucleic acid), CeNA (Cyclohexene nucleic acid), tcDNA (Tricyclo-DNA), 2'O-methyl etc., or mixtures thereof.

The nucleic acid polymer according to the invention may also be selected from artificially synthesized polymers similar to DNA or RNA, such as for example morpholino or PMO (Phosphorodiamidate Morpholino Oligo), PNA (Peptidic Nucleic Acid), 2'-5' linked oligonucleotides, etc.

In a particular embodiment, Y is a PNA.

### Applications of conjugates (I) and (X)-(XV)

The conjugates according to the invention having formulae (I) or (X)-(XV), and particularly the conjugates according to the invention having formulae (X)-(XV), have numerous applications in the field of modifying the level of expression of genes.

The invention thus relates to a method for modifying the level of expression of a gene in a cell, said method comprising the step of contacting said cell with a sufficient amount of a flavin-NAL conjugate having the formulae (I), (X), (XI), (XII), (XIII), (XIV) and (XV), wherein Y is complementary to a significant portion of the sequence of the RNA encoded by said gene.

The invention also relates to a flavin-NAL conjugate having the formulae (I), (X), (XI), (XII), (XIII), (XIV) and (XV), for use in a method for modifying the level of expression of a gene in a cell, said method comprising the step of contacting said cell with a sufficient amount of said flavin-NAL conjugate, wherein, in said flavin-NAL conjugate, Y is complementary to a significant portion of the sequence of the RNA encoded by said gene.

Particularly, the invention relates to a method for modifying the level of expression of a gene in a cell *in vitro,* said method comprising the step of contacting said cell with a sufficient amount of conjugate according to the invention selected from the group consisting of the conjugates having the formulae (I), (X), (XI), (XII), (XIII), (XIV) and (XV), wherein Y is complementary to a significant portion of the sequence of the RNA encoded by said gene.

In the method for modifying the level of expression of a gene in a cell *in vitro* according to the invention, during said step of contacting said cell with a conjugate according to the invention, the internalization of the conjugate by the cell can be facilitated by any transfection method known by the skilled person, such as for example electroporation, microinjection, etc.

The invention also relates to a method for modifying the level of expression of a gene in a cell *in vivo,* said method comprising the step of administering to an organism in which the expression of said gene shall be modified, a sufficient amount of a conjugate according to the invention selected from the group consisting of the conjugates having the formulae (I), (X), (XI), (XII), (XIII), (XIV) and (XV), wherein Y is complementary to a significant portion of the sequence of the RNA encoded by said gene. Typically, said sufficient amount is an amount effective for modifying the expression of said gene.

The invention also relates to a conjugate according to the invention selected from the group consisting of the conjugates having the formulae (I), (X), (XI), (XII), (XIII), (XIV) and (XV), for use in a method for modifying the level of expression of a gene in a cell *in vivo,* wherein Y is complementary to a significant portion of the sequence of the RNA encoded by said gene.

According to the invention, said RNA encoded by said gene is either a mRNA, a functional RNA, such as for example a rRNA, a tRNA, or a micro-RNA, or any non-coding RNA.

Accordingly, the conjugates according to the invention are useful either for research work or in therapy, for instance for treating diseases wherein a gene is overexpressed.

In a particular embodiment of said methods for modifying the level of expression of gene in a cell, the conjugate which is used has the following formula (G):

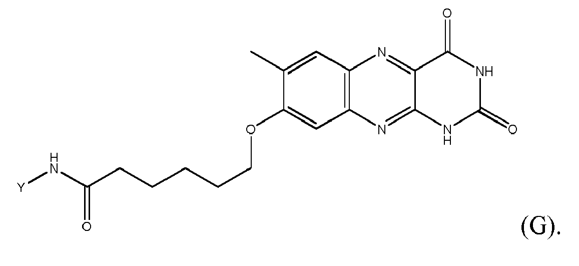

### Methods for obtaining the conjugates according to the invention

The invention also relates to a method for producing a flavin-NAL conjugate according to the invention, said method comprising the step of reacting a NAL with a compound having the formula (1) 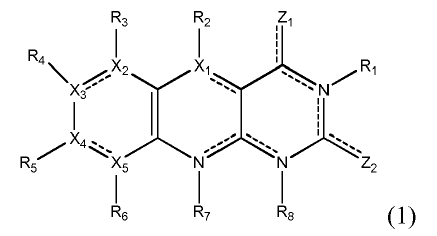 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂ₒ linear or branched and substituted or not alkyl,
- Z₂ is selected from the group consisting of [-A-L-], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, Cₗ-C₂₀ linear or branched and substituted or not alkyl, CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅, R₆, R₇ and R₈ are independently present or not and if present are independently selected from the group consisting of [-A-L-], H, F, Cl, Br, I, C₁-C₂₀ linear or branched and substituted or not alkyl, CF₃, -NH₂, -O-NH₂, - NHR₉, -O-NHR₉, -NR₉R₁₀, -O-NR₉R₁₀, -COOH, -CO-NH₂, -COO-NH₂, - COO-R₉, -CHO, -OH, -O-R₉, and -CO-R₉, wherein R₉ and R₁₀ represent independently a C₁-C₂₀ linear or branched and substituted or not alkyl,
wherein only one of Z₂, R₅, R₆, R₇ and R₈ represents [-A-L-], and wherein
- A is selected from the group consisting of =CH-, -CH₂-, -O-, -S-, -NH-, and
- L represents a linker comprising a terminal chemical reactive group Z capable of reacting with said NAL to form a conjugate wherein the A and the Y moieties are separated by at least 3 consecutive atoms.

Y is as defined previously in formula (I).

All the particular embodiments disclosed previously for formula (I) apply *mutatis mutandis* for formula (1) and are therefore not repeated therein.

Typically, said chemical reactive group Z is selected from the group consisting of any functional group capable of binding by a covalent bond directly or after activation, to at least one of the functions naturally present or artificially introduced onto Y. By way of non-limitative examples of reactive chemical functions Z appropriate to the purposes of the invention, there can be mentioned in particular the functions carboxylic acid and its salts, sulphonic acid and its salts, acid anhydride, acid chloride, ester (alkyl ester, p-nitrophenyl ester, succinimidyl ester, sulphosuccinimidyl ester, etc.), azido (acyl azide, azidonitrophenyl, etc.), hydrazide, 3-acyl-1,3-thiazolidine-2-thione, amine, substituted amine, O-alkyl hydroxylamine, quaternary ammonium, isocyanate, isothiocyanate, hydrazine, phthalimido, maleimide, haloacetamide, monochlorotriazine, dichlorotriazine, mono- or dihalogenated pyridine, mono- or dihalogenated diazine, aziridine, thiol, sulphonyl chloride, vinylsulphone, disulphide, methanethiosulphonate, hydroxyl, phosphoramidite, epoxy, aldehyde, carbonate, glyoxal, imidazolyl, alkyne, phosphate, H-phosphonate, thiophosphate, phosphoramidate.

Once Y and the compound having formula (1) have reacted together, a conjugate according to the invention is formed, wherein the NAL and the flavin moiety are conjugated via a linker L according to the invention, said linker L comprising the remainder of the chemical reactive group which has reacted with Y. This remainder of the chemical reactive group Z is typically the T₂ moiety according to the invention.

Synthesis of the compounds having formula (1) according to the invention can be performed using classical methods known by the skilled person. Examples of methods for synthesizing the compounds according to the invention are for instance disclosed by Simon et al., Angew. Chem. Int. Ed., 45, 6859-6861; Schwogler et al., Organic Letters, 200, Vol.2, No.10, 1415-1418; Hisafumi et al., Tetrahedron Letters, 42(2001), 2529-2531. If necessary, these methods can be easily adapted by the skilled person to synthesize the different compounds according to the invention, as for instance described in the experimental section.

The invention further relates to conjugates obtainable by the method for producing a flavin-NAL conjugate according to the invention, and to their applications as described previously.

Further aspects and advantages of this invention will be disclosed in the following figures and examples, which should be regarded as illustrative and not limiting the scope of this application.

### FIGURES

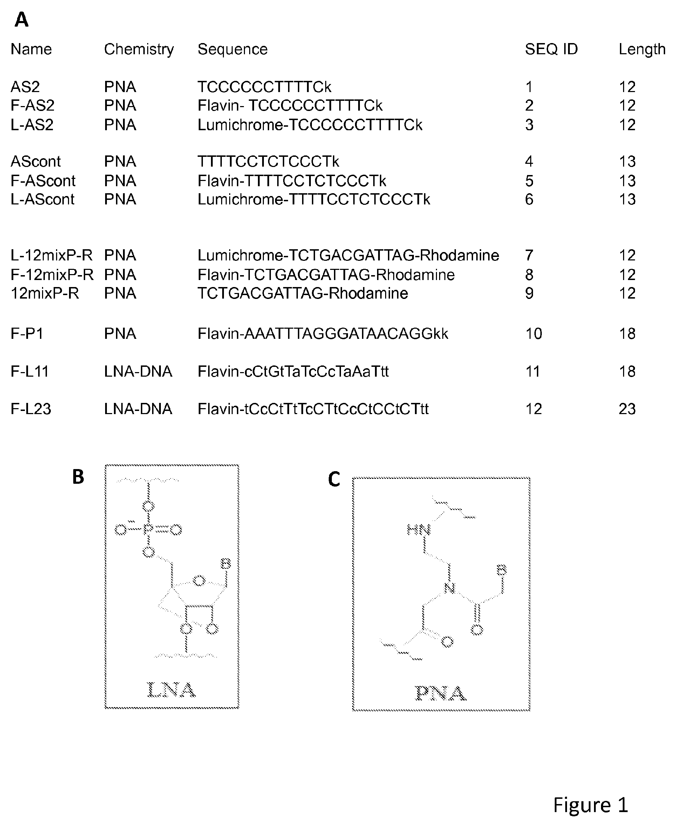

**Figure 1****.** Sequences of LNA conjugates and PNA conjugates.
   **A)** Names, chemistry, sequences, length of different LNAs and PNAs used for conjugation with flavin or lumichrome ("k" stands for a lysine). Note that Lumichrome is a structural analogue of flavin, but it is not redox. Thus, no DNA cleavage can be targeted/induced using conjugates with lumichrome. In LNA-DNA oligonucleotides, capital letters refer to DNA nucleotides, small letters to LNA nucleotides. **B)** LNA nucleotide structure. **C)** PNA monomer structure.

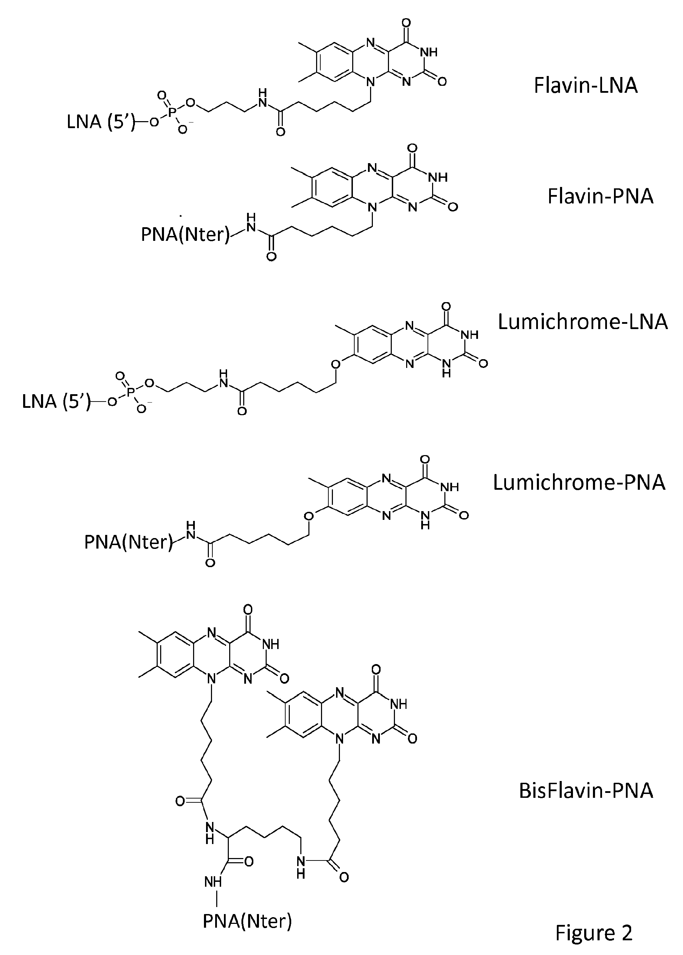

**Figure 2****.** Chemical structures of LNA conjugates and of PNA conjugates. Flavin and lumichrome were conjugated to the 5' end of LNA-containing oligonucleotides and to the N-terminal end of PNAs. A bis-flavin derivative was also conjugated to the N-terminal end of PNAs.

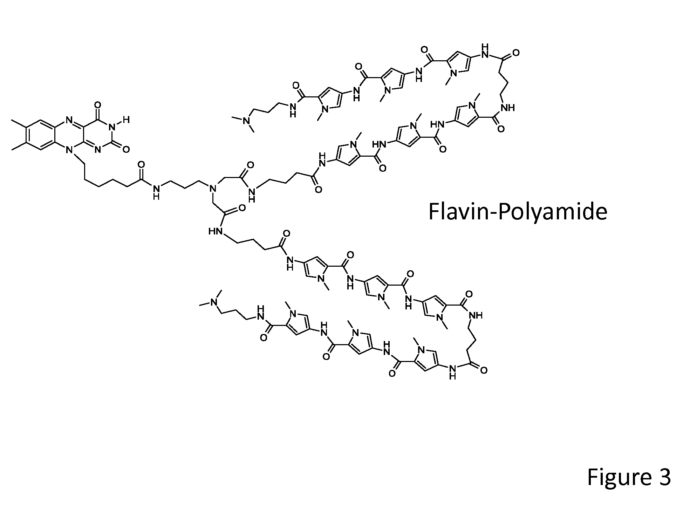

**Figure 3****.** Chemical structure of polyamide conjugates. The indicated conjugate is composed of two polyamides of 6 *N*-methylpyrrole.

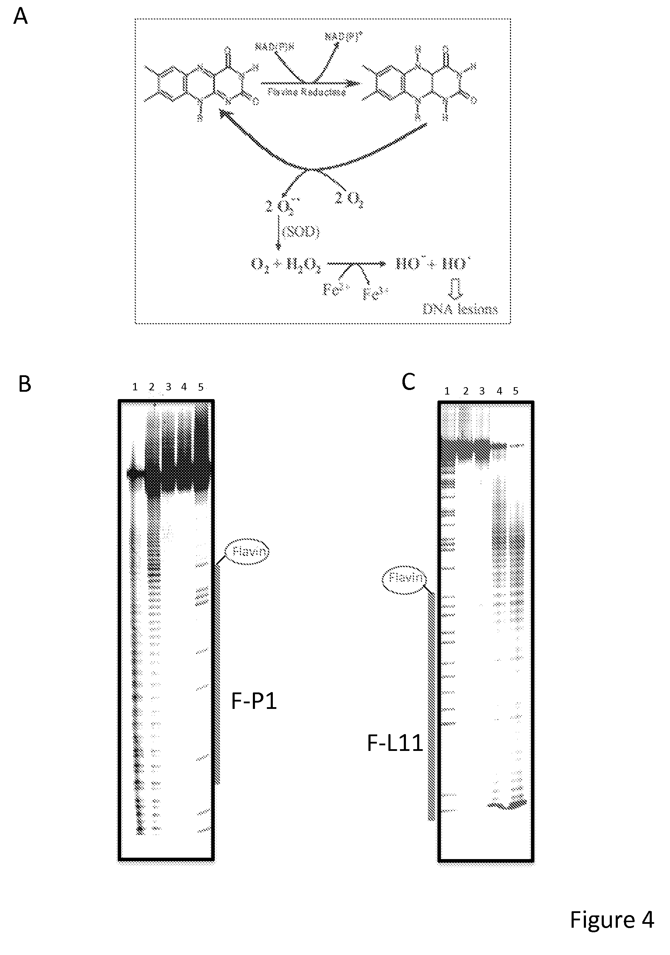

**Figure 4****.** Flavin conjugate cleavage activity *in vitro.* **A)** Potential mechanism for generation of hydroxyl radicals by flavin conjugates. The reaction implies the presence of a flavin reductase, ferric ions and molecular oxygen. **B, C)** Targeted cleavage activity of flavin-PNA (FP1, **B)** or flavin-LNA (F-L11, **C)** conjugate on 57 nt long DNA targets, resp., in the presence of flavin reductase, FRE. The target site of the flavin conjugates are schematically described near the gels. B) Lane 1: free riboflavin+DNA+FRE+NADH+Fe(II)+SOD; lane 2: FP1+DNA+FRE +NADH+Fe(II)+SOD; lane 3: DNA+FRE+NADH+Fe(II)+SOD; lane 4: DNA+FP1; lane 5: sequencing products G+A. C) Lane 1: sequencing products G+A; lane 2: DNA+F-L1; lane 3: DNA+ FRE+NADH+Fe(II)+SOD; lane 4: DNA +F-Ll+ FRE+NADH+Fe(II)+SOD; lane 5: DNA+ free riboflavin+FRE+NADH+Fe(II)+SOD.
**Figure 5****.** Cleavage activity of flavin conjugates in cells. 10µlM of the F-L23 flavin-LNA conjugate was added directly to the culture medium of mouse (53BP1-GFP-10T1/2) cells and observations of 53BP1-GFP foci were performed 40h after treatment. Foci abundance suggests F-L23 is able to penetrate into cells. Flavin conjugate is also able to induce DSBs in the cellular genome when directly added to the culture medium.
**Figure 6****.** Specific inhibition of gene expression by flavin- and lumichrome-PNA conjugates. CMV(+)PPT/HeLa cells were transfected with PNAs (AS2 and AScont) conjugated or not to flavin and lumichrome using SLO permeabilization. Luciferase activity was measured 24h after treatment.
**Figure 7****.** Lumichrome-PNA delivery into HeLa cells. 1µM of L-12mixP-R or F-12mixP-R or 12mixP-R were incubated in OptiMEM for 4h and then in DMEM with 10% FCS.
   Fluorescence microscopy was performed 4, 24 and 48 hours after addition of rhodamine conjugates. Strong fluorescence localized into cells is clearly visible with L-12mixP-R or F-12mixP-R while almost no fluorescence is detectable with 12mixP-R, demonstrating that cellular delivery is mediated by the lumichrome or flavin moieties.

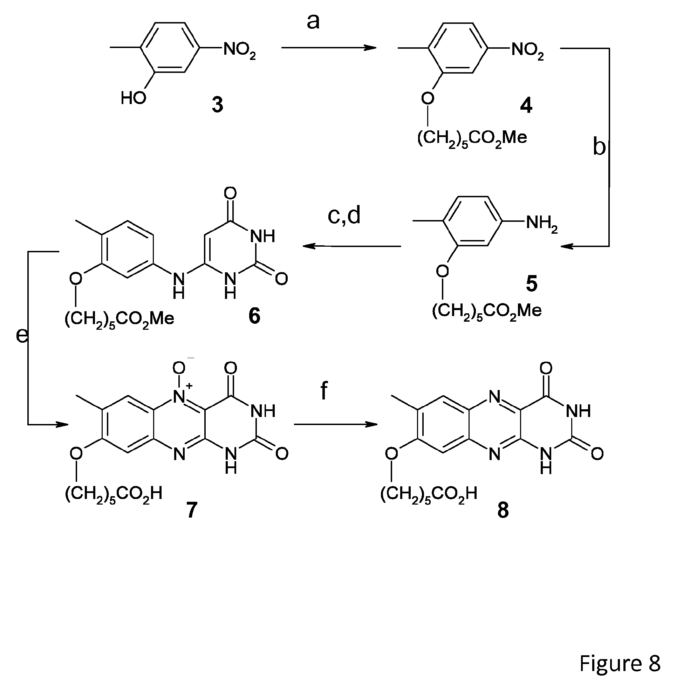

**Figure 8****.** Synthesis of a lumichrome derivative according to the invention

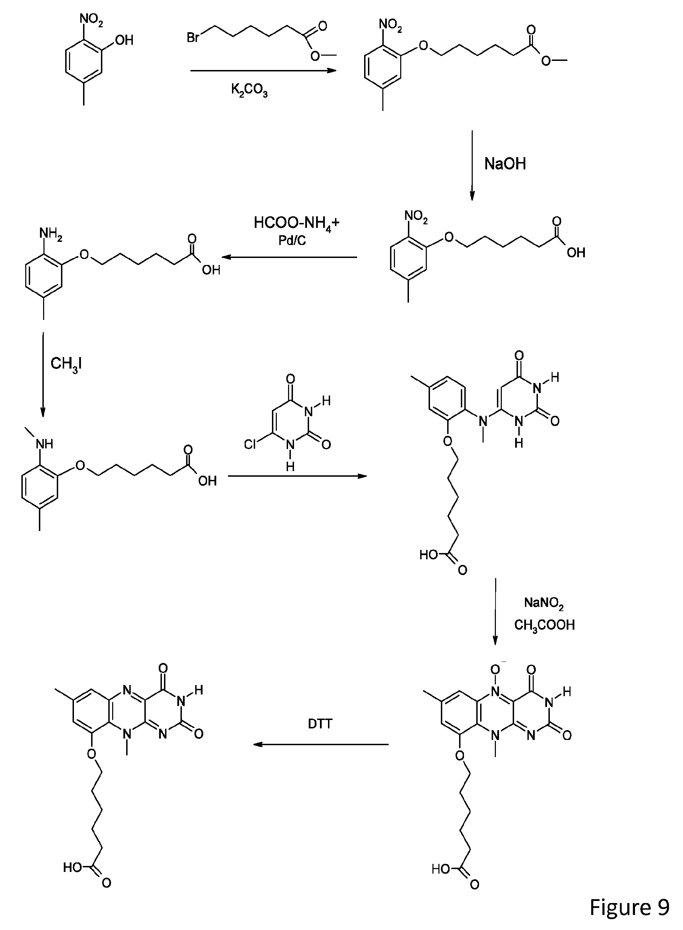

**Figure 9****.** Synthesis of a flavin conjugate according the invention having redox activity, substituted by the linker in position 9 of the isoalloxazine ring.

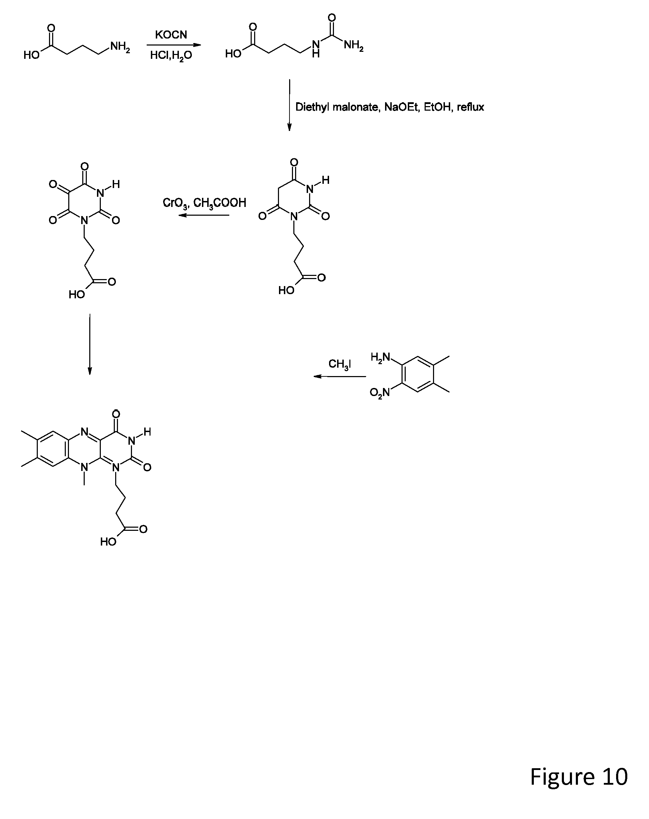

**Figure 10****.** Synthesis of a flavin conjugate according the invention having redox activity, substituted by the linker in position 1 of the isoalloxazine ring.

### EXAMPLES

### Results

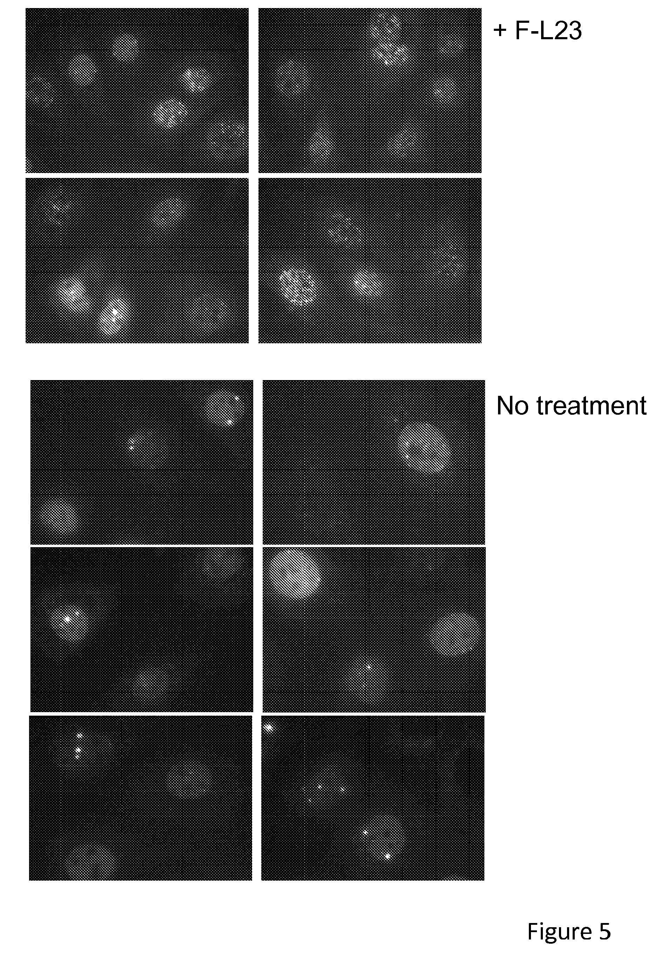

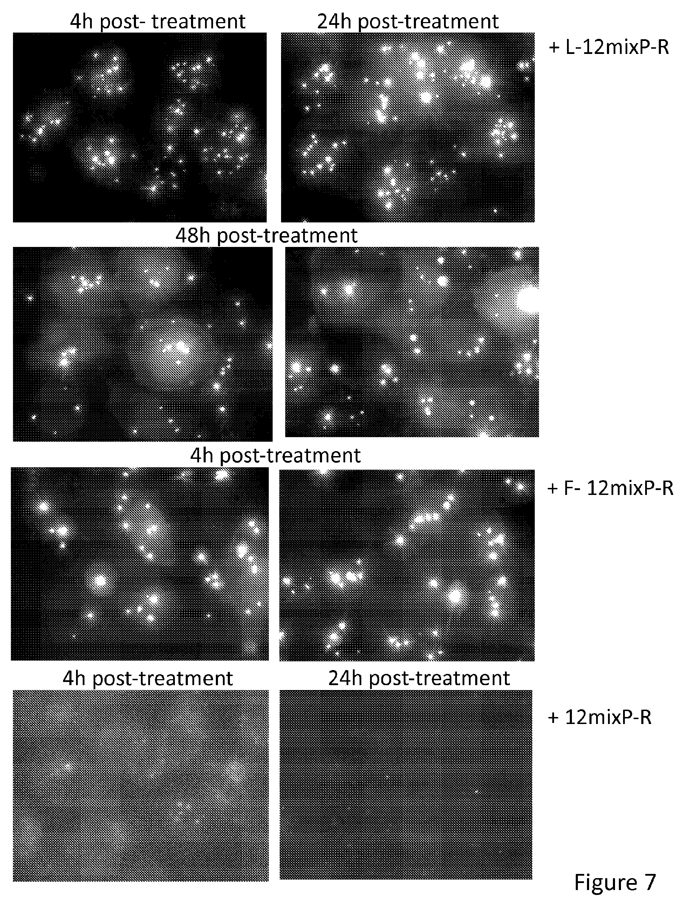

### Cell penetration properties of the conjugates according to the invention (Fig. 5 & 7)

An interesting and unexpected property of flavin and lumichrome is their ability to mediate cellular uptake of the entire conjugate.

Results obtained using lumichrome-PNA-rhodamine conjugates show that these molecules enter in cell nuclei within 24 hours from addition to the culture medium (Fig. 7) whereas no fluorescence was detected with the PNA-rhodamine conjugate. The same type of observations were performed using a flavin-PNA-rhodamine conjugate: 24 hours after addition of to culture medium a strong fluorescence was visible inside cells. Thus, lumichrome and flavin might represent an interesting approach for overcoming the extremely low efficiency of PNA penetration into cells. In addition since flavin has been described to enter specifically in some cell-types, its conjugation to nucleic acid analogues should permit to exhibit some targeted uptake properties.

Interestingly, flavin-LNAs seem to share the same penetration properties than flavin-PNAs. In this case we evaluated flavin-LNA uptake properties by a functional test based on nuclease activity of flavin conjugates (Fig.5 see details below). Nuclease activity of flavin-LNA conjugate FL23 was observed following direct addition to the cell culture medium, supporting uptake capacity.

### Nuclease activity- Targeting flavin to specific DNA sequences as a way of generating site-specific DNA damage (Fig. 4 & 5)

The nuclease activity of flavin-PNA and flavin-LNA conjugates was evaluated *in vitro* on a DNA target. In the presence of the flavin reductase enzyme, NADH and iron, DNA cleavage was observed specifically at proximity of the conjugate target site where the flavin is located (Fig. 4). The cleavage occurred on around 10 nt, consistent with the generation of diffusible OH° radicals following Fenton reaction. The nuclease activity of flavin-PNA is consistent with the already published data (Simon et al, 2006, Angewandte Chem.). In contrast the nuclease activity of flavin-LNA conjugate is unexpected since flavin-phosphodiester conjugates were previously reported to be unable to recruit the flavin reductase enzyme (Simon et al, 2005, Angewandte Chem;Thèse de Cécile Dueymes). The apparent discrepancy is likely due to structural differences between complexes formed with LNA or DNA on the complementary DNA sequence.

Cellular cleavage activity of flavin-LNA conjugate was evaluated by induction of repair foci in GFP-53BP1-10T1/2 cells (Fig.5). Indeed, it is well known that following break induction repair proteins are rapidly recruited to the lesion and for example, 53BP1 foci are induced. The cells were treated by the conjugate and observed under the microscope 48 hours after treatment. 53BP1-GFP foci were strongly induced compared to untreated cells. In addition the fluorescence pattern is highly different to the one observed with the free riboflavin (data not shown), consistent with a DNA sequence targeting property of the conjugate.

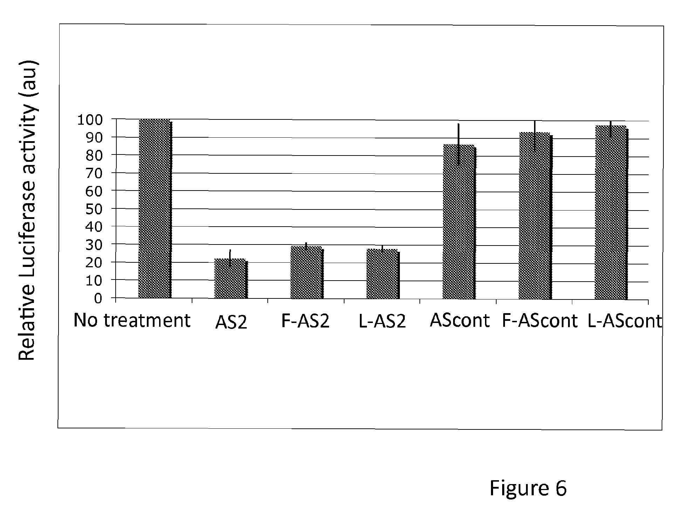

### Conjugate-mediated modulation of gene expression (Fig. 6)

In order to investigate the capacity of flavin-PNA and lummichrome-PNA conjugates to modulate gene expression, conjugates were transfected into human cell lines by streptolysin-O (SLO) mediated permeabilization. To address this question we used a reporter system: the CMV(+)PPT/HeLa cells stably contained the firefly luciferase gene (luc) under the control of a doxycycline-inducible CMV promoter and the conjugate target sequence (5'-AAAAGAAAAGGGGGGA-3', SEQ ID NO:15) was present in the 5' transcribed but untranslated region of the luciferase gene, downstream of the transcription start site. Cells were treated by a 12-mer PNA (AS2) conjugated or not to flavin and lumichrome and luciferase expression measured 24 hours later: around 70% inhibition of luciférase expression was observed in the présence of AS2, flavin-AS2 and lumichrome-AS2. In contrast a control PNA sequence (AScont) lacking a target site in the reporter sequence did not exhibit any effect nor the flavin-AScont or the lumichrome-AScont. These data demonstrate a sequence-specific gene inhibition mediated by flavin and lumichrome PNA conjugates.

### Discussion

The inventors have significantly improved the power of the oligonucleotide-based approaches. They have described a novel family of conjugate between flavin and nucleic acid ligands that can penetrate cells and exhibit nuclease activity. Penetration properties of conjugates with flavin and lumichrome (not redox) concern uncharged PNA and also negatively charged oligonucleotides (like LNA). In addition since flavin and lumichrome are known to enter specific cell-types, likely via receptor-mediated uptake (Yonezawa et al, 2008, Am J Physiol Celle Physiol; Yamamoto et al, 2009, J Biochem), their conjugation to NAL might allow targeted delivery which is of high interest when therapeutic applications are envisionned.

Targeted nuclease activity of the flavin conjugate is based on the ability of NAL to position flavin to specific sequence sites maintaining its redox activity. Such properties could be exploited in basic research especially in the DNA repair field or in genome modification applications.

This study confirms that the flavin and lumichrome conjugation strategy has a general validity and it could open the road to future developments of these molecules as a new and attractive class of modulators of gene expression and sequence-specific synthetic nucleases with intrinsic penetration properties.

### Experimental procedures

### Synthesis of a Lumichrome conjugate according the invention (Fig. 8)

### 6-(2-Methyl-5nitro-phenoxy)-hexanoic acid methyl ester 4:

To a solution of 2-methyl-5-nitrophenol 3 (3.9 g ; 25.5 mmol) in DMF (20 mL) refluxed under Argon, potassium carbonate (17.6 g ; 127.5 mmol) and 6-bromohexanoic acid methyl ester (8 g ; 38.3 mmol) were added with stirring. The mixture was refluxed for 30 min and was filtered after cooling. After evaporation, the residue was dissolved in dichloromethane and the solution was extracted three times by water. After purification by column chromatography on silica gel, the organic layer was dried over Na₂SO₄ and evaporated. The compound 4 was isolated (6.97 g ; 24.8 mmol ; 97.3 %). Mp: 47 °C; ¹H NMR (300 MHz, CDCl₃) δ 7.71 (1H, dd, ArH), 7.60 (1H, s, ArH), 7.22 (1H, d, ArH), 4.03 (2H, t, CH₂O), 3.66 (3H, s, COOCH3), 2.34 (2H, t, CH₂COO), 2.27 (3H, s, CH₃), 1.85 (2H, m, 2CH₂), 1.71 (2H, m, 2CH₂), 1.53 (2H, m, CH₂). MS (ESI⁺) m/z : 282 ([M+H]⁺).

### 6-(5-Amino-2-methyl-phenoxy)-hexanoic acid methyl ester 5:

A mixture of compound 4 (6 g ; 21.4 mmol) and palladium on charcoal 5% (120 mg) was refluxed in methanol (40 mL). Ammonium formate (12.2 g ; 193.6 mmol) was added and the reaction mixture was stirred at 55°C for 1 hour. After cooling, the mixture was filtered and reduced under vacuum. The residue was dissolved in diethylether and the solution was extracted three times by water. After purification by column chromatography on silica gel, the compound 5 was obtained after evaporation (2.68 g ; 10.7 mmol ; 50 %). Mp: 94 °C; ¹H NMR (300 MHz, CDCl₃) δ 6.86 (1H, d, ArH), 6.17 (2H, ms ArH), 3.88 (2H, t, CH₂O), 3.65 (3H, s, COOCH3), 3.47 (2H, s, ArNH₂), 2.34 (2H, t, CH₂COO), 2.07 (3H, s, CH₃), 1.79 (2H, m, 2CH₂), 1.70 (2H, m, 2CH₂), 1.51 (2H, m, CH₂). MS (ESI⁺) m/z : 252 ([M+H]⁺).

### Uracil derivative 6:

To a solution of compound 5 (2.3g, 9.3 mmol) in 1,4-dioxanne/water (1:1, 20 mL) refluxed under argon, 6-chlorouracil (1 g, 6.9 mmol) was added with stirring. The mixture was refluxed for 48 h increasing at regular intervals pH near to 7.5 by addition of aqueous NaOH 3M. After cooling, aqueous NaOH was added until pH 13 and the solution was stirred for 8 h and then extracted with dichloromethane. Compound 6 was precipitated from the aqueous solution by addition of 6M aqueous hydrochloric acid to reach pH 1. After filtration and washing with water and, then, diethylether, compound 6 was isolated (2.14 g, 6.2 mmol, 66.2 %). Mp: 207 °C;¹H NMR (300 MHz, DMSO-d6) δ 10.35 (1H, s, NH), 8.20 (1H, m, NH), 7.10 (1H, d, ArH), 6.73 (1H, s, ArH), 6.67 (1H, d, ArH), 4.65 (1H, s, CH), 3.95 (2H, t, CH₂O), 2.22 (2H, t, CH₂COO), 2.11 (3H, s, CH₃), 1.73 (4H, m, 2 CH₂), 1.56 (2H, m, CH₂), 1.46 (2H, m, CH₂). MS(ESI⁺) m/z : 348 ([M+H]⁺).

### N-oxide 7:

Sodium nitrite (560 mg, 8.2 mmol) was added in the dark to a solution of compound 6 (1.5g, 4.3 mmol) in acetic acid (10 mL) cooled in an ice bath. The mixture was stirred at room temperature for 5 h, and then water (2 mL) was added. The suspension was stirred again for 3 h, and then was evaporated to dryness. After addition of water and filtration, the solid was washed with water and then diethylether. Compound 7 was obtained as a yellow powder (0.79 g, 2.1 mmol, 92.5 %). Mp: 261 °C (dec); ¹H NMR (300 MHz, DMSO-d6) δ 11.74 (1H, s, NH), 11.57 (1H, s, NH), 8.11 (1H, s, ArH), 7.10 (1H, s, ArH), 4.20 (2H, t, CH₂O)_{,} 2.38 (3H, s, CH₃), 2.25 (2H, t, CH₂COO), 1.84 (2H, m, 2 CH₂), 1.62 (2H, m, CH₂), 1.52 (2H, m, CH₂). MS(ESI⁺) m/z : 375 ([M+H]⁺).

### Lumichrome 8:

Dithiothreitol (1,38 g, 9 mmol) was added to a solution of N-oxide 7 (0.61 g, 1.63 mmol) in DMF (60 ml). The mixture was refluxed with stirring for 12 h under argon. The cooled solution was stirred at room temperature for 10 h and then evaporated to dryness. The residue was dissolved in a minimum volume of aqueous 3M NaOH. Aqueous 6M HCl was added for precipitation of compound 8. After filtration and washing with water and then diethylether, pure compound 8 was obtained (530 mg, 1.5 mmol, 90%). Mp 252 °C (dec); ¹H NMR (300 MHz, DMSO-d6) δ 11.95 (1H, s, COOH), 11.74 (1H, s, NH), 11.57 (1H, s, NH), 7.89 (1H, s, ArH), 7.16 (1H, s, ArH), 4.20 (2H, t, CH₂O), 2.35 (3H, s, CH₃), 2.25 (2H, m, CH₂COO), 1.83 (2H, m, 2 CH₂), 1.60 (2H, m, CH₂), 1.51 (2H, m, CH₂). MS (ESI⁺) m/z : 359 ([M+H]⁺).

The lumichrome thus obtained can be conjugated with a NAL according to the invention.

### Synthesis of a flavin conjugate according the invention having redox activity, substituted by the linker in position 9 of the isoalloxazine ring (Fig. 9)

The first step involves the Williamson reaction between the 5-methyl-2-nitrophenol and the bromoester. After hydrolysis of the ester function by NaOH, the nitro group is reduced by HCOO⁻NH4⁺ in presence of Pd on charcoal and then alkylated by methyl iodide. The resultant product is condensed with the 6-chlorouracil and then cyclized with NaNO₂ in acetic acid. Reduction of the N-oxide is then effectued by treatment with dithiothreitol to give the modified flavin which have a linker with a carboxylic acid function on position 9.

### Synthesis of a flavin conjugate according the invention having redox activity, substituted by the linker in position 1 of the isoalloxazine ring (Fig. 10)

The first step is the formation of the ureido function by reaction of the 4-aminobutiric acid with isocyanate following by hydrolysis. Second step involves a malonic synthesis and lead to the modified barbituric acid which is then oxidized in an alloxan derivative with CrO₃. The resultant alloxan then condensed with an aromatic amine to give the desired modified flavin which has a linker with a carboxylic acid function on position 1. The aromatic amine is obtained easily by reaction of 4,5-Dimethyl-2-nitroaniline with methyl iodide, followed by the reduction of the nitro group with HCOO⁻NH4⁺ in presence of Pd on charcoal.

### Synthesis of a Lumichrome-PNA conjugate according to the invention

To a solution of lumichrome 8 described above (4 mg, 11 µmol) in DMF (2 mL), DIPEA (0.1 mL) and HATU (O-(7-azabenzotriazol-1-yl)-N,N,N',N'-tetramethyluronium hexa-fluorophosphate; 4 mg , 11 µmol) were added under argon. The resulting solution was stirred for 15 min and, then, was added under argon to the protected PNA on the solid support (PNA synthesis at 2 µM scale). The mixture was stirred at room temperature for 1 h and filtered. The beads were washed with DMF and, then, diethyl ether. The flavin-PNA conjugate was cleaved from the solid support with concomitant deprotection by treatment with TFA/m-cresol (3:1) for 1 h. The mixture was filtered and, then, diethyl ether was added to the solution to precipitate the lumichrome-PNA conjugate. HPLC purification was carried out on a C₁₈ reversed-phase X-bridges column (Waters, 10 x 250 mm). Elution was performed at 60°C by building up the following gradient at a flow rate of 2 mL min⁻¹ from 0.1% aqueous TFA to 0.1% TFA in acetonitrile (detection at 290 and 350 nm). MS (ESI+) m/z: calcd 3897.5, found 3897.7.

***Synthesis of bis-flavin-PNA conjugates.*** The bisflavin-PNA was synthetized as described previously for the PNA-flavin (Simon et al, Angewandte, 2006) using a PNA on solid support with a deprotected lysine on the N-terminal.

***Synthesis of flavin-LNA conjugates.*** Concerning LNA-flavin conjugates, all chemical reagents were obtained from Aldrich and ACROS Organics. Flavin with an acid carboxylic fonction was synthesized using a method previously reported (Simon et al Angewandte, 2006). LNA were purchased from Eurogentec (Seraing, Belgium) on solid support with a C3 amino linker on the 5' end.

Diisopropylamine (0.1 mL) and HATU (*O*-(7-azabenzotriazol-1-yl)-*N,N,N;N*'-tetramethyl uronium hexa-fluorophosphate; 4 mg , 11 µmol) were added under argon to a solution of flavin (4 mg, 11 µmol) in dimethylformamide (2 mL). The resulting solution was stirred for 15 min and, then, was added under argon to the protected LNA on the solid support (LNA synthesis at 1 µM scale). The mixture was stirred at room temperature for 1 h and filtered. The beads were washed with DMF and, then, diethyl ether. The flavin-LNA conjugate was cleaved from the solid support with concomitant deprotection by treatment with concentrated aqueous ammonia in methanol:water 50:50 for 12 h. The mixture was filtered and then evaporated under vacuum. After evaporation, water was added and the LNA-flavin conjugates were purified solubilized in water and purified on a C₁₈ reversed-phase Atlantis column (Waters, 10 x 250 mm). Elution was performed at 50°C with a gradient of acetonitrile (from 0 to 35% in 35 min) in 10 mM ammonium acetate buffer (pH 7) at a flow rate of 2 ml/min (detection at 260 and 450 nm). LNA-flavin conjugates were analysed by electrospray ionization mass spectrometry (ESI-MS) analyses.

***In vitro cleavage assay.*** Two 57 nt long oligonucleotides,
5'GCAAATTTAGGGATAACAGGCCTATTAAGCCGTAACCTGCTGAGTTAC TGCTAGGTT-3' (SEQ ID NO:13) or 5'-GTACAATTACCCTGTTATCCCTAAATTTGCCTCTCCCCCCTTTTCTTTTAA AGCTTG-3' (SEQ ID NO:14), were used as targets (1µM) and incubated with 0.5 µM flavin conjugate (F-L11 or F-P1, resp.) in the presence of NADH (5mM), FeS04 (80µM), superoxide dismutase (SOD, 0.13 mg/ml, from Sigma-Aldrich) and FRE (1 µM) in 10 mM phosphate buffer pH 7.8, as indicated. The reaction was carried out at 37°C during 90 min. The réaction mixture was analyzed by denaturing PAGE. The enzyme used here is the flavin reductase from *E.coli* named FRE prepared as previously described (Nivière et al, 1998, JBiolChem).

***Cell cultures.*** The CMV(+)PPT/HeLa cells were derived from HeLa/Tet-on cells (Clontech) that are engineered HeLa cells, stably expressing rTet protein necessary for the expression of doxycycline-inducible promoters. The CMV(+)PPT/HeLa cells stably contained two reporter genes, the firefly luciferase (Photinus pyralis) gene (luc) and the GFP gene, under the control of a bidirectional doxycycline-inducible CMV promoter as described in Brunet et al, Nucleic Acids Res. (2006) 34, 4546-4553. Importantly, a 55 bp insert containing the wild-type HIV-1 polypurine tract target sequence (PPT: 5'-AAAAGAAAAGGGGGGA-3', SEQ ID NO:15) was present in the 5' transcribed but untranslated region of the luciferase gene, downstream of the transcription start site.

The GFP-53BP1-10T1/2 cell line was generated from HeLa cells stably transfected by the full-length mouse 53BP1 fused to GFP. The details of the construction have been described previously (Jullien et al, 2002, J Cell Sci, 115, 71-79). Neomycin-resistant cells were cloned. Clones were screened using camptothecin treatment and observation Under the microscope. Clones producing green nuclear foci in around 90% cells were further propageted and one of them was used in this study.

CMV(+)PPT/HeLa cells and GFP-53BP1-10T1/2 cells were grown in 75 cm² plastic flasks in DMEM containing 10% FBS at 37°C in a 5% CO₂ atmosphere. Media changes were done every 2 days.

***Cell imaging of 53BP1 foci.*** 30000 GFP-53BP1-10T1/2 cells were plated on a 8 well Lab-Tek chamber slide (Thermo Scientific) with 10µM of F-L23 conjugate in 200 µl of DMEM+10% FBS. 40h after treatment, cells were observed in HBSS with a Leica DMIRE2 fluorescence microscope.

***Cell permeabilization.*** Streptolysin-O (SLO; a gift from S.Bhakdi, Johannes Gutenberg-Universitat, Mainz, Germany) was used to reversibly permeabilize HeLa cells. The protocol used has already been described elsewhere (Brunet et al, Nucleic Acids Res. (2006) 34, 4546-4553). Briefly, cells were washed twice in HBSS without Ca2+ (GIBCO) and 2.6 x10⁵ cells were resuspended in 20µl HBSS. An optimized amount of SLO and 2 µl of 10x PNA (*i.e*. 10 µM) were added for a l5min incubation at 37° to allow SLO-induced permeabilization. 180µl of DMEM containing Ca²⁺ were added to reseal cells for 30min and cells were then seeded in triplicate in a 96 well plate. Permeabilization efficiency was controlled by flow cytometry 1 hour after addition of DMEM. Permeabilization yield was typically about 80%.

***Firefly luciferase assay.*** 2 hours after SLO permeabilization, 1 gg/ml doxycycline were added to induce luciferase expression and 24 hours after SLO permeabilization, cells were washed once with PBS and lysed with Passive Lysis Buffer (Promega). Cell lysates were analysed with Luciferase Assay System (Promega) and relative Firefly luciferase light units were read using a Wallac Victor² system. The relative light units were normalized to the absorbance data of a Bradford protein assay (Bio-Rad) also read in a Wallac Victor² system.

***Fluorescent imaging of PNA conjugate uptake.*** 22000 HeLa cells were plated on a µslide 8 well (Ibidi) in 200µl of DMEM+10% FBS and cultured overnight. The medium was discarded and cells were washed with PBS followed by incubation with 150µl of 1µM Lumichrome-PNA-Rhodamine or Flavin-PNA-Rhodamine or PNA-Rhodamine, in OptiMEM for 4h. Cells were then washed with PBS and 200µl of HBSS were added to the cells for observation with a Leica DMIRE2 fluorescence microscope.

## Claims

1. A flavin-NAL conjugate having formula (I) 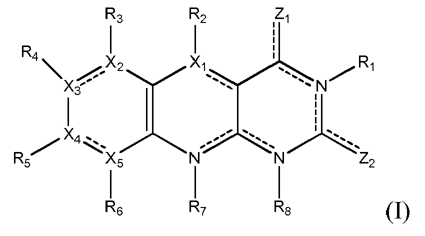 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂₀ linear or branched and substituted or not alkyl,
- Z₂ is selected from the group consisting of [-A-L-Y], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, -NH₂, -CF₃, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅, R₆, R₇ and R₈ are independently present or not and if present are independently selected from the group consisting of [-A-L-Y], H, F, Cl, Br, I, C₁-C₂₀ linear or branched and substituted or not alkyl, -NH₂, -CF₃, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂ₒ linear or branched and substituted or not alkyl,
wherein only one of Z₂, R₅, R₆, R₇ and R₈ represents [-A-L-Y], and wherein
- A is selected from the group consisting of =CH-, -CH₂-, -O-, -S-, -NH-,
- L is a linker which separates A and Y by a chain of at least 3 consecutive atoms, and
- Y is a Nucleic Acid Ligand (NAL).

2. The flavin-NAL conjugate according to claim 1, wherein said NAL is selected from the group consisting of:
- a single or double strand nucleic acid polymer comprising from 5 to 200 nucleotides or a synthetic pyrrole-imidazole polyamide composed of 2 to 15 units, and
- a DNA or RNA antisense oligonucleotide comprising from 5 to 200 nucleotides,
said NAL being capable of pairing with a target DNA or RNA sequence.

3. A flavin-NAL conjugate having formula (II) 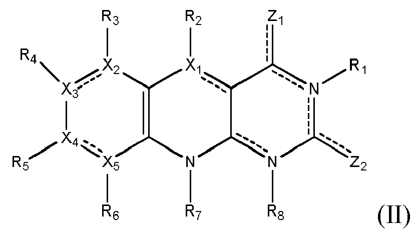 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂₀ linear or branched and substituted or not alkyl,
- Z₂ is selected from the group consisting of [-A-L-Y], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂ₒ linear or branched and substituted or not alkyl, and
- R₅, R₆, and R₈ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
- R₇ is selected from the group consisting of [-CH₂-L-Y] and C₁-C₂₀ linear or branched and substituted or not alkyl,
wherein only one of Z₂, R₅, R₆, R₇ and R₈ represents [-A-L-Y] or [-CH₂-L-Y], and wherein
o A is selected from the group consisting of =CH-, -CH₂-, -0-, -S-, - NH-,
o L is a linker which separates A and Y by a chain of at least 3 consecutive atoms, and
o Y is a NAL.

4. The flavin-NAL conjugate according to claim 3, wherein said NAL is a single or double strand nucleic acid polymer comprising from 5 to 200 nucleotides or a synthetic pyrrole-imidazole polyamide composed of 2 to 15 units, said NAL being capable of pairing with a target DNA or RNA sequence.

5. The flavin-NAL conjugate according to claim 4, wherein said NAL is a TFO (Triplex Forming Oligonucleotide).

6. The flavin-NAL conjugate according to any one of claims 4-5, wherein said NAL comprises one or more nucleic acid analogues in its sequence or is constituted exclusively of nucleic acid analogues, said nucleic acid analogues being preferably selected from the group comprising GNA (Glycol Nucleic Acid), LNA (Locked Nucleic Acid), TNA (Threose Nucleic Acid), Phosphorothioates, Propyne analogs, N3'-P5' phosphoroamidate, FANA (2'-fluoro-arabino nucleic acid), CeNA (Cyclohexene nucleic acid), tcDNA (Tricyclo-DNA), 2'0-methyl, and mixtures thereof.

7. The flavin-NAL conjugate according to claim 4, wherein said NAL is an artificially synthesized polymer similar to DNA or RNA, said artificially synthesized polymer similar to DNA or RNA being preferably selected from the group comprising morpholino or PMO (Phosphorodiamidate Morpholino Oligo), PNA (Peptidic Nucleic Acid) and 2'-5' linked oligonucleotides.

8. A flavin-NAL conjugate having formula (X) 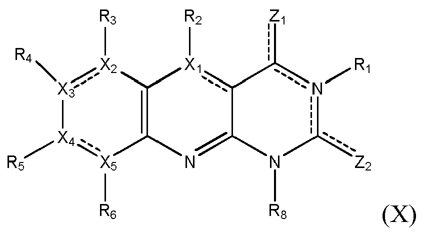 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂ₒ linear or branched and substituted or not alkyl,
- Z₂ is selected from the group consisting of [-A-L-Y], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂ₒ linear or branched and substituted or not alkyl, and
- R₅, R₆ and R₈ are independently present or not and if present are independently selected from the group consisting of [-A-L-Y], H, F, Cl, Br, I, C₁-C₂₀ linear or branched and substituted or not alkyl, -CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl,
wherein only one of Z₂, R₅, R₆ and R₈ represents [-A-L-Y], and wherein
- A is selected from the group consisting of =CH-, -CH₂-, -O-, -S-, -NH-,
- L is a linker which separates A and Y by a chain of at least 3 consecutive atoms, and
- Y is a Nucleic Acid Ligand (NAL).

9. The flavin-NAL conjugate according to claim 8, wherein said NAL is a DNA or RNA antisense oligonucleotide comprising from 5 to 200 nucleotides, said NAL being capable of pairing with a target RNA sequence.

10. The flavin-NAL conjugate according to any one of claims 9, wherein said NAL comprises one or more nucleic acid analogues in its sequence or is constituted exclusively of nucleic acid analogues, said nucleic acid analogues being preferably selected from the group comprising GNA (Glycol Nucleic Acid), LNA (Locked Nucleic Acid), TNA (Threose Nucleic Acid), Phosphorothioates, Propyne analogs, N3'-P5' phosphoroamidate, FANA (2'-fluoro-arabino nucleic acid), CeNA (Cyclohexene nucleic acid), tcDNA (Tricyclo-DNA), 2'0-methyl, and mixtures thereof.

11. The flavin-NAL conjugate according to claim 9, wherein said NAL is an artificially synthesized polymer similar to DNA or RNA, said artificially synthesized polymer similar to DNA or RNA being preferably selected from the group comprising morpholino or PMO (Phosphorodiamidate Morpholino Oligo), PNA (Peptidic Nucleic Acid) and 2'-5' linked oligonucleotides.

12. A method for transfecting a cell with a Nucleic Acid Analogue (NAL), said method comprising the step of contacting said cell with a flavin-NAL conjugate, said flavin-NAL conjugate being preferably selected from the group consisting of the flavin-NAL conjugates as defined in any one of claims 1-11.

13. A flavin-NAL conjugate for use in a method for transfecting a cell with a NAL, said method comprising the step of contacting said cell with a flavin-NAL conjugate, said flavin-NAL conjugate being preferably selected from the group consisting of the flavin-NAL conjugates as defined in any one of claims 1-11.

14. A method for modifying a target DNA sequence in a cell, said method comprising the step of contacting said cell with a sufficient amount of a flavin-NAL conjugate as defined in any one of claims 3-7 wherein Y is complementary to a significant portion of the target DNA sequence.

15. The flavin-NAL conjugate as defined in any one of claims 3-7, for use in a method for modifying a target DNA sequence in a cell, said method comprising the step of contacting said cell with a sufficient amount of said flavin-NAL conjugate, wherein, in said flavin-NAL conjugate, Y is complementary to a significant portion of the target DNA sequence.

16. A method for modifying the level of expression of a gene in a cell, said method comprising the step of contacting said cell with a sufficient amount of a flavin-NAL conjugate as defined in any one of claims 3-11 wherein Y is complementary to a significant portion of the sequence of the RNA encoded by said gene.

17. The flavin-NAL conjugate as defined in any one of claims 3-11, for use in a method for modifying the level of expression of a gene in a cell, said method comprising the step of contacting said cell with a sufficient amount of said flavin-NAL conjugate, wherein, in said flavin-NAL conjugate, Y is complementary to a significant portion of the sequence of the RNA encoded by said gene.

18. A method for producing a flavin-NAL conjugate according to the invention, said method comprising the step of reacting a NAL with a compound having the formula (1) 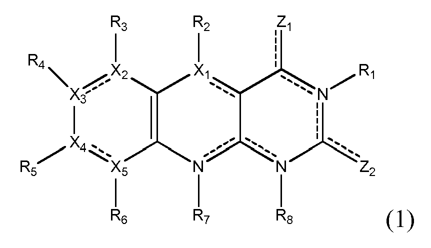 wherein
- the doted lines represent a bond which is present or not,
- X₁, X₂, X₃, X₄ and X₅ are independently selected from the group consisting of C, CH or N,
- Z₁ is selected from the group consisting of =O, =S, -O-R₉ and -S-R₉, wherein R₉ represents a C₁-C₂ₒ linear or branched and substituted or not alkyl,
- Z₂ is selected from the group consisting of [-A-L-], =O, =S, -O-R₉ and -S-R₉, wherein R₉ represents a C₁-C₂₀ linear or branched and substituted or not alkyl,
- R₁, R₂, R₃ and R₄ are independently present or not and if present are independently selected from the group consisting of H, F, Cl, Br, I, C₁-C₂₀ linear or branched and substituted or not alkyl, CF₃, -NH₂, -O-NH₂, -NHR₉, -O-NHR₉, -NR₉R₁₀, -O-NR₉R₁₀, -COOH, -CO-NH₂, -COO-NH₂, -COO-R₉, -CHO, -OH, -O-R₉, and -CO-R₉, wherein R₉ and R₁₀ represent independently a C₁-C₂₀ linear or branched and substituted or not alkyl, and
- R₅, R₆, R₇ and R₈ are independently present or not and if present are independently selected from the group consisting of [-A-L-], H, F, Cl, Br, I, C₁-C₂₀ linear or branched and substituted or not alkyl, CF₃, -NH₂, -O-NH₂,-NHR₉, -O-NHR₉, -NR₉R₁₀, -O-NR₉R₁₀, -COOH, -CO-NH₂, -COO-NH₂,-COO-R₉, -CHO, -OH, -O-R₉, and -CO-R₉, wherein R₉ and R₁₀ represent independently a C₁-C₂₀ linear or branched and substituted or not alkyl,
wherein only one of Z₂, R₅, R₆, R₇ and R₈ represents [-A-L-], and wherein
- A is selected from the group consisting of =CH-, -CH₂-, -O-, -S-, -NH-, and
- L represents a linker comprising a terminal chemical reactive group Z capable of reacting with said NAL to form a conjugate wherein the A and the Y moieties are separated by at least 3 consecutive atoms.
